(19)

**Europäisches Patentamt**
**European Patent Office**
**Office européen des brevets**

(11) **EP 4 606 418 A1**

(12) **EUROPEAN PATENT APPLICATION**
published in accordance with Art. 153(4) EPC

(43) Date of publication:
**27.08.2025 Bulletin 2025/35**

(21) Application number: **23879074.5**

(22) Date of filing: **16.10.2023**

(51) International Patent Classification (IPC):
**A61N 1/362** (2006.01)

(52) Cooperative Patent Classification (CPC):
**A61N 1/362; A61N 1/365; A61N 1/39**

(86) International application number:
**PCT/CN2023/124791**

(87) International publication number:
**WO 2024/083085 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.10.2022 CN 202211265014**

(71) Applicant: **United Innomed (Shanghai) Limited Shanghai 201315 (CN)**

(72) Inventor: **WANG, Li**
**Shanghai 201315 (CN)**

(74) Representative: **Banse & Steglich**
**Patentanwälte PartmbB**
**Patentanwaltskanzlei**
**Herzog-Heinrich-Straße 23**
**80336 München (DE)**

(54) **CARDIAC THERAPY APPARATUS**

(57)     The present application relates to a cardiac therapy apparatus. The cardiac therapy apparatus comprises: a subcutaneous defibrillation assembly, which comprises a first control component and is configured to sense a global activation event of a heart, generate a far-field sense signal indicative of the global activation event, and deliver a defibrillation wave to a patient when the patient has malignant arrhythmia; and a leadless myocardial stimulation assembly, which comprises a second control component and is configured to sense a local activation event of myocardium at a predetermined stimulation position, generate a local sense signal indicative of the local activation event, and apply to the predetermined stimulation position an electrical stimulation impulse for enhancing the myocardial contractility, wherein the first control component is coupled to the second control component through wireless communication, and the first control component and/or the second control component are/is configured to determine, at least according to the far-field sense signal and the local sense signal, whether to apply the electrical stimulation impulse to the predetermined stimulation position.

FIG. 1

EP 4 606 418 A1

**Description**

FIELD OF THE INVENTION

The present application relates to the field of cardiac therapy, and more specifically, to an implantable multifunctional cardiac therapy apparatus.

BACKGROUND

Cardiac contractility modulation (CCM) is a unique and innovative therapy for the treatment of patients with chronic heart failure. It enhances the contractility of the myocardium by applying an electrical stimulus to the cardiac muscle, thereby achieving therapeutic purposes. Unlike conventional pacemakers or cardiac resynchronization therapy devices, the electrical stimulus delivered by CCM devices is applied during the absolute refractory period and does not affect the heart rate or action potential distribution. Conventional CCM devices available on the market generally include a main body that is implanted outside the heart and two bipolar leads extending into an interventricular septum of a ventricle to detect electrical activities of local cardiac muscle in the ventricle and the temporal sequence of the electrical activities, determine a delivery time period for applying an electrical impulse stimulus to the cardiac muscle, and applying the electrical impulse stimulus to local myocardial cells in the delivery time period to enhance the contractility of the cardiac muscle.

However, the two bipolar leads of the conventional CCM device that are implanted in the body affect the normal blood flow in the blood vessel and right ventricle and the closure of the tricuspid valve, and may fall off and cause infection (including capsular bag, electrode leads, etc.), leading to additional safety risks. In addition, because the conventional CCM device determines the delivery time period of the electrical impulse stimulus based on an electrical signal at the local cardiac muscle, the determined delivery time period may be incorrect. For example, if the CCM device determines that the electrical signal at the local cardiac muscle corresponds to the R-wave of the cardiac cycle, but in fact the signal corresponds to the T-wave or other event (e.g., an interference signal), the electrical impulse stimulus applied by the CCM device will act on the cardiac muscle during the T-wave phase, leading to a significantly increased risk of ventricular tachycardia (VT) or ventricular fibrillation (VF) associated with malignant ventricular arrhythmias.

In addition, given the same energy, a larger distance between stimulation electrodes or a larger effective area of the stimulation electrodes indicates a higher degree and/or a larger range of far-field simulation of cardiac muscle. If a local impulse stimulus occurs at the part where myocardial cells are depolarized at a later moment, the time of application of the electrical impulse stimulus may be within the supernormal phase of the action potential of myocardial cells at the cardiac muscle where myocardial cells are depolarized earlier, which may lead to re-depolarization of this part of cardiac muscle. To avoid the above problems, conventional CCM devices require stimulation electrodes to be arranged in the ventricular septum, and therefore are not suitable for use at other positions where enhanced contractility of cardiac muscle may be required. Moreover, to avoid the above risks, a control strategy for conventional CCM devices is to not apply an electrical impulse stimulus in the case of a changed in cardiac muscle action potential which is not caused by atrial (ectopic ventricular activation). However, the incidence of premature ventricular contraction in patients with heart failure is very high, and some patients with heart failure rely on ventricular pacing for a long time, especially for patients having taken beta blockers for a long time, implantable cardioverter-defibrillator (ICD) patients, and cardiac resynchronization therapy (CRT) patients. Therefore, conventional CCM devices cannot provide a desirable treatment effect for these patients. Furthermore, conventional CCM devices do not provide ICD treatment. Many patients have to choose between the two therapies, for example, due to economic reasons, and cannot get the best treatment. Although an CCM device and an ICD device can be implanted in the body at the same time, the patient has to face an, increased financial burden and the risk of complications.

On the other hand, some patients with heart disease currently use a subcutaneous implantable cardioverter-defibrillator (S-ICD), which provides safe and effective protection against sudden cardiac arrest. However, such devices do not bring any therapeutic value to patients in most cases, and can function only the when patient experiences ventricular tachycardia (VT) or ventricular fibrillation (VF) associated with life-threatening malignant ventricular arrhythmia.

In summary, it is necessary to provide a novel cardiac therapy apparatus and method to solve at least one of the problems in the prior art.

SUMMARY

An objective of the present application is to provide a cardiac therapy apparatus and a cardiac treatment method based on the cardiac therapy apparatus.

A first aspect of the present application provides a cardiac therapy apparatus, including: a subcutaneous defibrillation assembly, configured to be implanted beneath the skin of a patient and including a first control component and a plurality of subcutaneous electrodes, where the first control component is configured to detect a global activation event of the heart of the patient through at least a part of the plurality of subcutaneous electrodes, generate a far-field sense signal indicative of the global activation event, and deliver a defibrillation wave to the patient through at least a part of the plurality of subcutaneous electrodes when the patient has malignant arrhythmia; and a leadless myocardial stimulation assembly, configured to contact a predetermined stimulation position of the heart of the patient and including a second control component and a plurality of stimulation assembly electrodes, where the second control component is configured to detect a local activation event of myocardium at the predetermined stimulation position through at least a part of the plurality of stimulation assembly electrodes, generate a local sense signal indicative of the local activation event, and apply to the predetermined stimulation position an electrical stimulation impulse for enhancing the contractility of myocardial cells in the heart of the patient through at least a part of the plurality of stimulation assembly electrodes, where the first control component is coupled to the second control component through wireless communication, and the first control component and/or the second control component are/is configured to determine, at least according to the far-field sense signal and the local sense signal, whether to apply the electrical stimulation impulse to the predetermined stimulation position.

A second aspect of the present application further provides a cardiac therapy apparatus, including: a subcutaneous defibrillation assembly, configured to be implanted beneath the skin of a patient and including a first control component and a plurality of subcutaneous electrodes, where the first control component is configured to sense a global activation event of the heart of the patient through at least a part of the plurality of subcutaneous electrodes, generate a far-field sense signal indicative of the global activation event, and deliver a defibrillation wave to the patient through at least a part of the plurality of subcutaneous electrodes when the patient has malignant arrhythmia; and a leadless myocardial stimulation assembly, configured to contact a predetermined stimulation position of the heart of the patient and including a second control component and a plurality of stimulation assembly electrodes, where the second control component is configured to apply, to the predetermined stimulation position through at least a part of the plurality of stimulation assembly electrodes, an electrical stimulation impulse for enhancing the contractility of myocardial cells in the heart of the patient, and the second control component is configured to apply, to the predetermined stimulation position through at least a part of the plurality of stimulation assembly electrodes, a pacing electrical impulse for adjusting the heart rate of the patient, and generate a pacing electrical impulse signal indicating the pacing electrical impulse applied, where the first control component is coupled to the second control component through wireless communication, and the first control component is configured to determine, at least according to the far-field sense signal and the pacing electrical impulse signal, whether to apply the electrical stimulation impulse to the predetermined stimulation position.

A third aspect of the present application further provides a cardiac therapy apparatus, including: a subcutaneous defibrillation assembly, configured to be implanted beneath the skin of a patient and including a first control component and a plurality of subcutaneous electrodes, where the first control component is configured to sense a global activation event of the heart of the patient through at least a part of the plurality of subcutaneous electrodes, generate a far-field sense signal indicative of the global activation event, and deliver a defibrillation wave to the patient through at least a part of the plurality of subcutaneous electrodes when the patient has malignant arrhythmia; and a leadless myocardial stimulation assembly, configured to contact a predetermined stimulation position of the heart of the patient and including a second control component and a plurality of stimulation assembly electrodes, where the second control component is configured to sense a local activation event of myocardium at the predetermined stimulation position through at least a part of the plurality of stimulation assembly electrodes, generate a local sense signal indicative of the local activation event, and apply to the predetermined stimulation position an electrical stimulation impulse for enhancing the contractility of myocardial cells in the heart of the patient through at least a part of the plurality of stimulation assembly electrodes, where the first control component is coupled to the second control component through wireless communication, and the first control component is configured to determine, at least according to the far-field sense signal, whether a specific activation event occurs in the heart, and the first control component and/or the second control component are/is configured to determine, at least according to a result of the determination of whether the specific activation event occurs, whether to apply the electrical stimulation impulse to the predetermined stimulation position.

A fourth aspect of the present application further provides a subcutaneous implantable cardioverter-defibrillator, including: a housing, configured to be implanted under the skin of a patient; a plurality of electrodes, coupled to the housing and configured to be implanted the skin of the patient; a control module, coupled to a leadless heart failure treatment apparatus in contact with a predetermined stimulation position of the heart of the patient by wireless communication, to receive a local sense signal indicative of a local activation event of the myocardium at the predetermined stimulation position and generated by the leadless heart failure treatment apparatus based on sense of the local activation event, the control module being configured to sense a global activation event of the heart of the patient through at least a part of the plurality of electrodes, generate a far-field sense signal indicative of the global activation event, and determine, at least according to the far-field sense signal and the local sense signal, whether the local activation event of myocardial cells at the predetermined stimulation position corresponds to a specific global activation event or determine, at least according to the far-field sense signal and/or the local sense signal, whether an electrical stimulation impulse to be applied by the leadless heart failure treatment apparatus falls in an absolute refractory period of the local activation event and/or a specific global activation event, to determine whether to use the leadless heart failure treatment apparatus to apply the electrical stimulation impulse to the predetermined stimulation position; and an impulse generation module, received within the housing, electrically coupled to at least a part of the plurality of electrodes, and configured to deliver a defibrillation wave for treating malignant arrhythmia to the patient through at least a part of the plurality of electrodes under control of the control module.

A fifth aspect of the present application further provides a method of controlling a cardiac therapy apparatus, including: the cardiac therapy apparatus includes a subcutaneous defibrillation assembly and a leadless myocardial stimulation assembly, the subcutaneous defibrillation assembly is configured to be implanted under the skin of a patient and includes a first control component and a plurality of subcutaneous electrodes, and the leadless myocardial stimulation assembly is configured to contact a predetermined stimulation position of the heart of the patient and includes a plurality of stimulation assembly electrodes and a second control component coupled to the first control component by wireless communication, the method including: sensing, by the first control component, a global activation event of the heart of the patient through at least a part of the plurality of subcutaneous electrodes, and generating a far-field sense signal indicative of the global activation event; sensing, by the second control component, a local activation event of myocardium at the predetermined stimulation position through at least a part of the plurality of stimulation assembly electrodes, and generating a local sense signal indicative of the local activation event; and determining, by the first control component and/or the second control component at least according to the far-field sense signal and the local sense signal, whether to apply the electrical stimulation impulse to the predetermined stimulation position.

A sixth aspect of the present application provides a non-volatile computer-readable storage medium, having a computer program stored thereon, where the computer program, when executed by a processor, causes the processor to implement the steps of the method of the fifth aspect of the present application.

It can be understood that the electrical stimulation impulse in the present invention are used to enhance the contractility of myocardial cells in the heart and is delivered during the absolute refractory period, and the pacing electrical impulse is used to pace the heart and is delivered outside the absolute refractory period.

The above is a summary of the present application, where details may be simplified, summarized, or omitted. Therefore, it should be understood by those skilled in the art that this section is merely illustrative and is not intended to limit the scope of the present application in any way. This summary section is neither intended to determine key or essential features of the claimed subject matter, nor is it intended to be used as an aid in determining the scope of the claimed subject matter.

BRIEF DESCRIPTION OF DRAWINGS

The above and other features of the present application will become more fully understood from the following specification and the appended claims when taken in conjunction with the accompanying drawings. It can be understood that these drawings depict only some embodiments of the present application and should not be construed as limiting the scope of the present application. The contents of the present application will be more clearly described in detail with reference to the accompanying drawings.

FIG. 1 is a schematic diagram of a cardiac therapy apparatus implanted in the body of a patient according to an embodiment of the present application;
FIG. 2A to FIG. 2C are respectively schematic diagrams of an implantable heart failure treatment apparatus according to three different embodiments of the present application;
FIG. 3A to FIG. 3C are respectively schematic diagrams of an implantable heart failure treatment apparatus according to three different embodiments of the present application;
FIG. 4A to FIG. 4B are respectively schematic diagrams of an implantable heart failure treatment apparatus according to two different embodiments of the present application;
FIG. 5 is a schematic flowchart of a method 600 of controlling a cardiac therapy apparatus according to an embodiment of the present application;
FIG. 6 illustrates specific sub-steps included in step 603 of the method 600 shown in FIG. 5 according to an embodiment;
FIG. 7 illustrates specific sub-steps included in step 603 of the method 600 shown in FIG. 5 according to another embodiment; and
FIG. 8 illustrates a method 700 of controlling a cardiac therapy apparatus in a pacing mode according to an embodiment of the present application.

DETAILED DESCRIPTION

In the following detailed description, reference is made to the accompanying drawings which form a part of the detailed description. In the accompanying drawings, like symbols generally represent like components unless the context indicates otherwise. The illustrative embodiments described in the detailed description, accompanying drawings, and claims are not intended to be limiting. Other embodiments may be employed and other changes may be made without departing from the spirit or scope of the subject matter of the present application. It can be understood that various configurations, substitutions, combinations, designs may be made to various aspects of the present application generally described in the present application and illustrated in the accompanying drawings, all of which explicitly constitute a part of the present application.

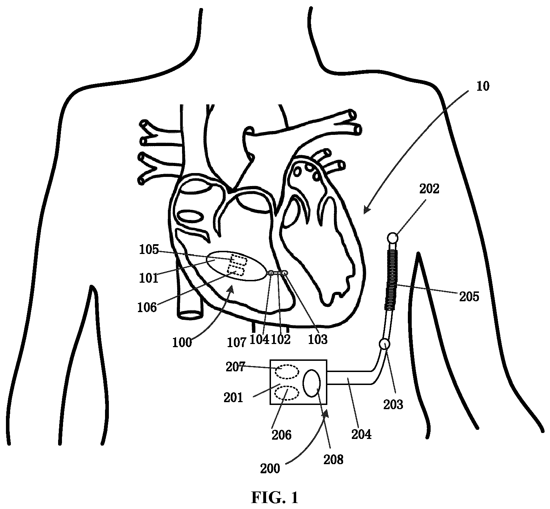

FIG. 1 is a schematic diagram of a cardiac therapy apparatus implanted in the body of a patient according to an embodiment of the present application. As shown in FIG. 1, the cardiac therapy apparatus 10 includes a subcutaneous defibrillation assembly 200 and a leadless myocardial stimulation assembly 100. The leadless myocardial stimulation assembly 100 is configured to contact a predetermined stimulation position of a heart of a patient. The subcutaneous defibrillation assembly 200 is configured to be implanted beneath the skin of the patient. To more clearly illustrate the product structure, the positions of the leadless myocardial stimulation assembly 100 and the subcutaneous defibrillation assembly 200 in FIG. 1 are schematically shown. The leadless myocardial stimulation assembly 100 may be arranged at any position in contact with the heart of the patient. The subcutaneous defibrillation assembly 200 may be arranged under the skin of the patient to sense a cardiac electrical signal of the patient and deliver a defibrillation wave to the patient when the patient has malignant arrhythmia. The specific positions of the leadless myocardial stimulation assembly 100 and the subcutaneous defibrillation assembly 200 will be described in detail below.

As shown in FIG. 1, the subcutaneous defibrillation assembly 200 includes a first control component 206, configured to control the subcutaneous defibrillation assembly 200 to sense a global activation event of the heart of the patient, generate a far-field sense signal indicative of the global activation event, and deliver a defibrillation wave to the patient when the patient has malignant arrhythmia. The leadless myocardial stimulation assembly 100 includes a second control component 106, which is coupled to the first control component 206 by wireless communication and is configured to control the leadless myocardial stimulation assembly 100 to sense a local activation event of myocardium at the predetermined stimulation position, generate a local sense signal indicative of the local activation event, and apply to the predetermined stimulation position an electrical stimulation impulse for enhancing the contractility of myocardial cells in the heart of the patient. It should be noted that, the "far-field sense signal" and the "local sense signal" herein may be signals generated based on sense of a global activation event or a local activation event and capable of indicating any relevant information of the global activation event or the local activation event. In some embodiments, the "far-field sense signal" and the "local sense signal" respectively include a moment at which the global activation event is sensed and a moment at which the local activation event is sensed.

The first control component 206 and/or the second control component 106 are/is configured to determine, at least according to the far-field sense signal and the local sense signal, whether and when to apply an electrical stimulation impulse for enhancing the contractility of myocardial cells in the heart of the patient to the predetermined stimulation position through the leadless myocardial stimulation assembly 100. In some embodiments, the first control component 206 and/or the second control component 106 are/is configured to determine whether to apply the electrical stimulation impulse to the predetermined stimulation position based on a determination result of at least one of the following two steps: i) determining, at least according to the far-field sense signal and the local sense signal, whether the local activation event of myocardial cells at the predetermined stimulation position corresponds to a specific global activation event; and ii) determining, at least according to the far-field sense signal and/or the local sense signal, whether the electrical stimulation impulse to be applied falls in an absolute refractory period of the local activation event and/or a specific global activation event.

Specifically, the first control component 206 and/or the second control component 106 may be configured to determine to apply the electrical stimulation impulse to the predetermined stimulation position, in response to determining that the local activation event (e.g., R-wave, where R-wave includes both an individual R-wave and a composite R-wave having a waveform morphology similar to a QRS complex) corresponds to a specific global activation event (e.g., R-wave or QRS complex, etc.). In some other embodiments, the first control component 206 and/or the second control component 106 are/is configured to determine to apply the electrical stimulation impulse to the predetermined stimulation position, in response to determining that the electrical stimulation impulse to be applied falls in an absolute refractory period of the local activation event and/or the specific global activation event, regardless of whether the local activation event corresponds to the global activation event. In some other embodiments, the first control component 206 and/or the second control component 106 are/is configured to determine to apply the electrical stimulation impulse to the predetermined stimulation position, in response to determining that the local activation event corresponds to a specific global activation event and that the electrical stimulation impulse to be applied falls in an absolute refractory period of the local activation event and/or the specific global activation event. For example, the first control component 206 may be configured to receive, from the second control component 106, the local sense signal indicative of the local activation event that is sensed by the leadless myocardial stimulation assembly 100. Then, the first control component 206 may determine, at least based on the far-field sense signal sensed by the subcutaneous defibrillation assembly 200 and the local sense signal sensed by the leadless myocardial stimulation assembly 100, whether the local activation event at the predetermined stimulation position corresponds to a specific global activation event, e.g., determine whether an electrical signal generated from action potentials of myocardial cell groups at and near the predetermined stimulation position corresponds to a specific far-field ECG change. In response to determining that the local activation event corresponds to the specific global activation event, the first control component 206 and/or the second control component 106 further determines, based on the far-field sense signal and/or the local sense signal, whether the electrical stimulation impulse to be applied falls in the absolute refractory period of the local activation event and/or the specific global activation event. In response to determining that the electrical stimulation impulse to be applied falls in the absolute refractory period of the local activation event and/or the specific global activation event, the first control component 206 and/or the second control component 106 determines to apply the electrical stimulation impulse for enhancing the contractility of myocardial cells in the heart of the patient to the predetermined stimulation position through the leadless myocardial stimulation assembly 100.

In some other embodiments, the first control component 206 and/or the second control component 106 may be configured to determine, at least according to the far-field sense signal, whether a specific activation event (e.g., R-wave or QRS complex, etc.) occurs in the heart, and determine, at least according to a result of the determination of whether the specific activation event occurs, whether to apply the electrical stimulation impulse to the predetermined stimulation position. Specifically, the first control component 206 and/or the second control component 106 may be further configured to: determine, according to the far-field sense signal and/or the local sense signal, whether the electrical stimulation impulse to be applied falls in the absolute refractory period of the local activation event and/or the specific global activation event, in response to determining based on the far-field sense signal that the specific activation event occurs; and determine to apply the electrical stimulation impulse for enhancing the contractility of myocardial cells in the heart of the patient to the predetermined stimulation position through the leadless myocardial stimulation assembly 100, in response to determining that the electrical stimulation impulse to be applied falls in the absolute refractory period of the local activation event and/or the specific global activation event. Specifically, in some embodiments, the first control component 206 determines whether a specific activation event (e.g., R-wave or QRS complex, etc.) occurs according to the sensed far-field sense signal indicative of the global activation event, and in response to determining that the specific activation event occurs, sends the determination result or an indication of delivering the electrical stimulation impulse to the second control component 106. After receiving the determination result or the instruction, the second control component 106 determines, according to the far-field sense signal and/or the local sense signal, whether the electrical stimulation impulse to be applied falls in the absolute refractory period of the local activation event and/or the specific global activation event; when the electrical stimulation impulse to be applied falls in the absolute refractory period of the local activation event and/or the specific global activation event, controls the leadless myocardial stimulation assembly 100 to apply the electrical stimulation impulse for enhancing the contractility of myocardial cells in the heart of the patient to the predetermined stimulation position; and when the electrical stimulation impulse to be applied does not fall in the absolute refractory period of the local activation event and/or the specific global activation event, skips an operation of applying the electrical stimulation impulse. Of course, in some embodiments, after receiving the determination result or the instruction, the second control component 106 may directly control the leadless myocardial stimulation assembly 100 to apply the electrical stimulation impulse for enhancing the contractility of myocardial cells of the heart of the patient to the predetermined stimulation position, without performing the above determination step.

Specifically, in some other embodiments, the first control component 206 and/or the second control component 106 are/is configured to determine whether to apply the electrical stimulation impulse to the predetermined stimulation position, when a determination result of at least one of the following two determination steps is "yes": i) determining, at least according to the far-field sense signal and the local sense signal, whether the local activation event of myocardial cells at the predetermined stimulation position corresponds to a specific global activation event; and ii) determining, at least according to the far-field sense signal and/or the local sense signal, whether the electrical stimulation impulse to be applied falls in an absolute refractory period of the local activation event and/or a specific global activation event.

Specific implementations of the above determination steps will be described in detail below. It should be noted that, as used herein, the phrases such as "the first control component and/or the second control component are/is configured to", "the first control component 206 and/or the second control component 106 are/is configured to", and other similar expressions are intended to mean that the specific steps, functions, and methods may be performed by either of the two control components alone, or that the two control components may respectively perform the specific sub-steps or sub-functions to finally achieve the steps, functions, and methods. Some parameters or signals needed by one control component to perform a step or sub-step may be acquired through communication with the other control component.

Specifically, in some embodiments, in response to determining that the local activation event corresponds to the specific global activation event, the first control component 206 further determines, based on the far-field sense signal and/or the local sense signal, whether the electrical stimulation impulse to be applied falls in the absolute refractory period of the local activation event and/or the specific global activation event. When the electrical stimulation impulse to be applied falls in the absolute refractory period of the local activation event and/or the specific global activation event, the first control component 206 sends an instruction of delivering the electrical stimulation impulse to the second control component 106. After receiving the instruction, the second control component 106 controls the leadless myocardial stimulation assembly 100 to apply the electrical stimulation impulse for enhancing the contractility of myocardial cells of the heart of the patient to the predetermined stimulation position. In some other embodiments, in response to determining that the local activation event corresponds to the specific global activation event, the first control component 206 may send intermediate processing data generated during the operation of determining whether the electrical stimulation impulse to be applied falls in the absolute refractory period of the local activation event and/or the specific global activation event to the second control component 106, and the second control component 106 continues to perform the remaining operation steps after receiving the intermediate processing data. In response to determining that the electrical stimulation impulse to be applied falls in the absolute refractory period of the local activation event and/or the specific global activation event, the second control component 106 controls the leadless myocardial stimulation assembly 100 to apply the electrical stimulation impulse.

In some other embodiments, in response to determining that the local activation event corresponds to the specific global activation event, the first control component 206 may send only the determination result to the second control component 106. The second control component 106 executes the step of determining whether the electrical stimulation impulse to be applied falls in the absolute refractory period of the local activation event and/or the specific global activation event; in response to determining that the electrical stimulation impulse to be applied falls in the absolute refractory period of the local activation event and/or the specific global activation event, controls the leadless myocardial stimulation assembly 100 to apply the electrical stimulation impulse; and in response to determining that the electrical stimulation impulse to be applied does not fall in or does not completely fall in the absolute refractory period of the local activation event, skips the operation of applying the electrical stimulation impulse. Specific methods of determining whether a local activation event corresponds to a specific global activation event and determining whether an electrical stimulation impulse to be delivered falls in an absolute refractory period of a local activation event and/or a specific global activation event will be described in detail below.

The subcutaneous defibrillation assembly 200 shown in FIG. 1 is a subcutaneous implantable cardioverter-defibrillator. The subcutaneous implantable cardioverter-defibrillator 200 includes a housing 201, a conductive electrode 208 arranged on a surface of the housing 201, a first control component 206 arranged in the housing, and a lead wire 204 extending out of the housing 201. Electrodes 202, 203, and 205 are arranged on the lead wire 204. The housing 201 may be implanted under the skin of a patient. In some embodiments, the housing 201 may be arranged on the chest cavity adjacent to the left armpit. The lead 204 is also implanted under the skin. In some embodiments, the lead 204 may extend beneath the skin from the housing 201 toward the xiphoid process of the patient and extend to the left side of the sternum, preferably parallel to and approximately 1 cm to 2 cm distant from the left side of the sternum. The electrodes 202 and 203 are a pair of subcutaneous detection electrodes that, together with the conductive electrode 208 on the housing 201, can define a plurality of vectors for detecting the overall electrical activity of the heart. The first control component 206 detects, through the pair of subcutaneous detection electrodes 202 and 203 in combination with the conductive electrode 208, a global activation event of the heart of the patient, e.g., a P-wave representing the time and potential changes of the depolarization of the atrial muscle group as a whole, a QRS complex representing the time and potential changes of the depolarization of the ventricular muscle group as a whole, a T wave representing the time and potential changes of the re-polarization of the ventricular muscle group as a whole, etc.

Although the electrodes 202 and 203 are respectively shown as a cap electrode and a ring electrode, the electrodes 202 and 203 may also be configured to have any other feasible structure as long as a global activation event can be detected. Preferably, the electrodes 202 and 203 are electrodes having a relatively large surface area, e.g., electrodes similar to electrodes for surface ECG monitoring body. As shown in FIG. 1, the electrode 205 is a coil electrode and forms a defibrillation electrode pair with the conductive electrode 208, so that a defibrillation wave for treating malignant arrhythmia can be delivered between the coil electrode 205 and the conductive electrode 208 under the control of the first control component 206. In some embodiments, the first control component 206 may also sense a global activation event of the heart through an electrode pair formed by the coil electrode 205 and the conductive electrode 208 on the housing 201. It should be noted that, the coil electrode 205 and the conductive electrode 208 may also be configured to have any other feasible structure. For example, the electrode 208 may be formed by part of the housing 201, and the coil electrode 205 may be placed under the left armpit of the patient, or under the left scapula of the back of the patient.

As shown in FIG. 1, the subcutaneous implantable cardioverter-defibrillator 200 further includes an impulse generation module 207. The impulse generation module 207 is received within the housing 201 and is electrically coupled to the coil electrode 205. When the first control component 206 senses tachyarrhythmia through a subcutaneous electrocardiogram from the detection electrodes 202 and 203 and the electrode 208, the impulse generation module 207 is configured to deliver a defibrillation wave to the patient through the coil electrode 205 and the conductive electrode 208 under the control of the first control component 206. It should be further noted that the subcutaneous defibrillation assembly 200 shown in FIG. 1 is merely an exemplary configuration, and other configurations may also be adopted. For example, in some embodiments, in addition to the lead wire 204, the subcutaneous defibrillation assembly 200 may also have one or more other lead wires and other electrode structures arranged on the other lead wires to detect a global activation event of the heart of the patient and/or deliver a defibrillation wave for treating tachyarrhythmia to the patient. In some embodiments, the detection electrode 203 may also have a suture hole configured to be sutured to a fixed position of tissue of the patient when implanted subcutaneously. In some embodiments, the subcutaneous defibrillation assembly 200 may be any conventional form of implantable cardiac defibrillation apparatus, or a combination of different components or elements of such implantable cardiac defibrillation devices.

Still referring to FIG. 1, the leadless myocardial stimulation assembly 100 is an implantable heart failure treatment apparatus. The implantable heart failure treatment apparatus 100 includes a housing 101 and an anchoring member 102 connected to the housing 101. The anchoring member 102 is configured to secure the housing 100 to a specific position in a heart of a patient. Although not specifically shown in the figures, the anchoring member 102 may include various suitable mechanical components, e.g., a pin portion, a staple portion, a screw portion, a hook portion, a threaded portion, a spiral portion, a toothed portion, a clamping portion, and/or other similar structures, so as to be secured to cardiac tissue. In some embodiments, one or more anchoring members 102 may have a hook portion configured to pierce cardiac tissue and be at least partially retained in the cardiac tissue. In some other embodiments, one or more anchoring members 102 may have an auger structure similar to a wine bottle opener, so as to be drilled into cardiac tissue and thus fixed. In some other embodiments, one or more anchoring members 102 may have a screw portion configured to be tightly engaged with cardiac tissue by spiral rotation. It can be understood by those skilled in the art that the structure of the anchoring member 102 is merely exemplary and not limiting. The implantable heart failure treatment apparatus 100 may be implanted in a body by various suitable means, such as transapical or transvascular implantation.

Although only one anchoring member 102 is shown in FIG. 1, the implantable heart failure treatment apparatus 100 may also have any other suitable number of anchoring members 102 to secure the housing 101 to cardiac tissue. For example, the implantable heart failure treatment apparatus 100 may include one, two, three, four, five, six, seven, eight, or more anchoring members 102. In addition, the implantable heart failure treatment apparatus 100 may also adopt a combination of the above-described anchoring member forms to achieve a more stable fixation effect.

Referring to FIG. 1, the implantable heart failure treatment apparatus 100 further includes a pair of stimulation electrodes including the electrodes 103 and 104 and coupled to the housing 100, and the electrode 103 or 104 may include one or more biocompatible electrically conductive materials, e.g., various metals or alloys known to be safe for implantation in a human body. The pair of stimulation electrodes are exposed to surrounding tissue and/or blood with which they are in contact, and therefore can send an electrical signal to and/or receive an electrical signal from the surrounding tissue and/or blood. The electrical signal may be conducted via cardiac tissue and/or blood. In some embodiments, the pair of stimulation electrodes 103 and 104 are configured to sense a local sense (or referred to as Local Sense (LS)) signal generated by discharge of myocardial cells at a predetermined stimulation position, and to apply an electrical stimulation impulse for enhancing the contractility of myocardial cells in the heart of the patient to the predetermined stimulation position.

Since the anchoring member 102 shown in FIG. 1 is implanted at, for example, the ventricular septum, the pair of stimulation electrodes 103 and 104 may apply an electrical stimulus to the ventricular septum nearby. However, depending on where the anchoring member 102 is located, the predetermined stimulation position at which the pair of stimulation electrodes 103 and 104 are located may be any other position in the heart to which the electrical stimulation impulse for enhancing the contractility of myocardial cells is to be applied. For example, the predetermined stimulation position may be the endocardium in the heart chamber of the patient, or a position on the epicardium of the heart of the patient. In some embodiments, the predetermined stimulation position corresponds to a part of ventricular tissue. In some other embodiments, the predetermined stimulation position may also be a part of atrial tissue. In some embodiments, the housing 101 of the device 100 is configured to be arranged in a venous vessel outside the heart of the patient (e.g., having a capsule-like structure), and the predetermined stimulation position is epicardial tissue adjacent to the coronary vein. In this case, the anchoring member 102 is also configured as a suitable structure. For example, the anchoring member 102 may be a stent, balloon, support arm, or other similar inflatable structure, or the anchoring member 102 may be an anchor or hook that extends out from the housing 101 and is in contact with the venous vessel wall. In some other embodiments, the anchoring member 102 may be formed by a part of the housing 101 and have a cross-sectional diameter configured to fit to a relatively narrow section of the venous vessel wall, thereby securing the housing 101 in the vessel.

As shown in FIG. 1, the implantable heart failure treatment apparatus 100 further has an impulse generation module 105 and a second control component 106 arranged in the housing 101 and electrically coupled to the pair of stimulation electrodes 103 and 104. Although the positions of the electrodes 103 and 104 as shown in the figures are on the anchoring member 102, the electrodes 103 and 104 may be any structure that can be electrically coupled to the impulse generation module 105 and the second control component 106 in the housing 101 and exposed to tissue and/or blood outside the housing 101. For example, at least one of the pair of electrodes 103 and 104 is not arranged on the anchoring member 102, such that when the anchoring member 102 is anchored to the predetermined stimulation position (e.g., endocardium), the electrode not arranged on the anchoring member 102 is not in contact with the myocardium. In some embodiments, the electrode 103 and/or the electrode 104 may be formed on an outer surface of the housing or arranged on other members extending from the housing. When the anchoring member 102 is secured to the heart or vascular tissue, the electrode 103 and/or the electrode 104 remains in contact with the heart or vascular tissue. For example, the anchoring member 102 may be configured to have a hook portion as previously described, and the electrode 103 and/or the electrode 104 is located on a projection extending from the housing and adjacent to the anchoring member 102. When the hook portion of the anchoring member 102 pierces cardiac tissue and is at least partially retained in the cardiac tissue, the projection on which the electrode 103 and/or the electrode 104 is located is in contact with the cardiac tissue. In some other embodiments, the electrode 103 and/or the electrode 104 may be arranged on or formed by an entirety or part of the anchoring member 102, and pierced or screwed into cardiac or vascular tissue along with the anchoring member 102. For example, the anchoring member 102 may have an auger structure similar to a wine bottle opener as previously described, where a part or an entirety of the auger constitutes the electrode 103 and/or the electrode 104, and the electrode 103 and/or the electrode 104 is at least partially screwed into cardiac tissue.

In some embodiments, the pair of stimulation electrodes 103 and 104 may also any electrode configuration for applying a pacing impulse in a conventional leadless pacemaker. In some embodiments, the electrode 103 and/or the electrode 104 may be defined by a part of the housing 101. For example, the surface of the housing 101 includes an insulating material, and a part of the housing 101 not covered by the insulating material constitutes the electrode 103 and/or the electrode 104. The electrode 103 and/or the electrode 104 may be formed by regions of any shape smaller than the surface area of the housing, e.g., circular, rectangular, annular, semi-annular, fan-shaped, and arched regions, etc. In some embodiments, the electrode 103 and/or the electrode 104 is configured to be partially located in the housing 101 and partially exposed from the surface of the housing 101. In some other embodiments, the electrode 103 and/or the electrode 104 is arranged on another member extending from the housing 101 and is spaced apart from the housing 101 and/or the anchoring member 102. It should be noted that the above description is not intended to limit the specific position or configuration of the electrode 103 and/or the electrode 104, and the electrodes 103 and 104 may adopt any combination of the above-described electrode position and configuration features, or may adopt any feasible positional arrangement or configuration that can achieve their functions.

As shown in FIG. 1, the electrode 103 in the pair of stimulation electrodes is configured to contact with or electrically connect to the predetermined stimulation position of myocardial tissue (which is the ventricular septum in the figure). The electrode 103 and/or the electrode 104 may be of any suitable size and/or shape. In some embodiments, the surface area of the electrode 103 for contact with the predetermined stimulation position is 1 $mm^2$ to 10 $mm^2$, preferably 1.5, 2, 3, 4, or 5 $mm^2$. In the pair of stimulation electrodes, the electrode 103 and the electrode 104 are spaced apart from each other. In some embodiments, the two electrodes are spaced apart by 2 mm to 20 mm, preferably 5 mm to 10 mm.

In the housing 101, the impulse generation module 105 is electrically coupled to the pair of stimulation electrodes 103 and 104 and is configured to generate an electrical stimulation impulse. The second control component 106 is electrically coupled to the pair of stimulation electrodes 103 and 104 and the impulse generation module 105, and is configured to sense a local activation event of myocardium at the predetermined stimulation position through the pair of stimulation electrodes 103 and 104, and generate a local sense signal indicative of the local activation event. When determining that the local activation event corresponds to a specific global activation event, the second control component 106 controls the impulse generation module 105 to deliver the electrical stimulation impulse to the pair of stimulation electrodes 103 and 104 in an absolute refractory period of the local activation event and/or the specific global activation event, thereby enhancing the contractility of myocardial cells of the myocardial cells during the local activation event to treat heart failure.

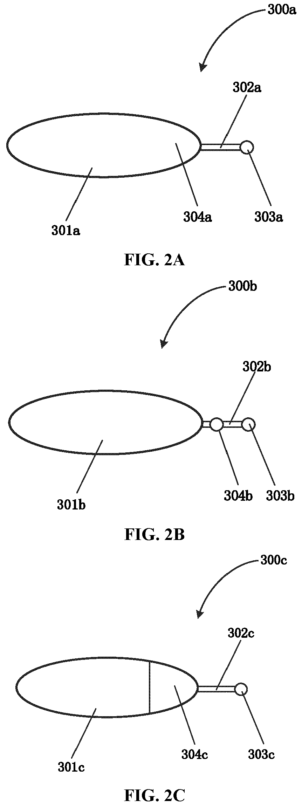

FIG. 2A to FIG. 2C are respectively schematic diagrams of implantable heart failure treatment apparatuses or leadless myocardial stimulation assemblies 300a, 300b, and 300c according to different embodiments of the present application, which schematically show alternative arrangements of the pair of stimulation electrodes relative to the housing.

As shown in FIG. 2A, the electrode 303a in the pair of stimulation electrodes of the apparatus 300a is located at an end of the anchoring member 302a distant from the housing 301a, and the other electrode 304a is arranged on an end of the housing 301a close to the anchoring member 302a. The arrangement of the pair of stimulation electrodes of the apparatus 300b shown in FIG. 2B is substantially the same as that in the embodiment shown in FIG. 2A, except that the stimulation electrode 304b is arranged on the anchoring member 302b, not on the housing 301b. The implantable heart failure treatment apparatus 300c shown in FIG. 2C differs from the implantable heart failure treatment apparatus 300b shown in FIG. 2B only in that the stimulation electrode 304b arranged on the anchoring member 302b is removed, and an end of the housing 301c close to the anchoring member 302c is configured as 304c.

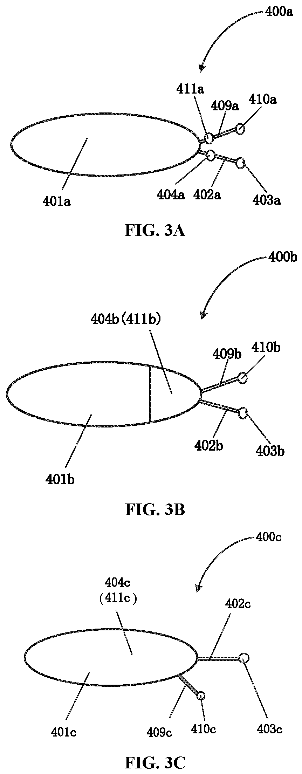

FIG. 3A to FIG. 3C are respectively schematic diagrams of implantable heart failure treatment apparatuses 400a, 400b, and 400c according to different embodiments of the present application, where each of the implantable heart failure treatment apparatuses includes a plurality of pairs of stimulation electrodes. The implantable heart failure treatment apparatus 400a shown in FIG. 3A has substantially the same structure as the implantable heart failure treatment apparatus 300b shown in FIG. 2B, except that the apparatus 400a further includes another anchoring member 409a, and another pair of stimulation electrodes 410a and 411a are arranged on the anchoring member 409a. The two pairs of stimulation electrodes are configured to respectively contact with different predetermined stimulation positions on the endocardium or epicardium. These pairs of stimulation electrodes can sense a local sense (LS) signal generated by the action potential of myocardial cells at the corresponding predetermined stimulation position, and apply an electrical stimulation impulses for enhancing the contractility of myocardial cells in the heart of the patient to the corresponding predetermined stimulation position.

In some embodiments, it is assumed that the electrode 403a in the pair of stimulation electrodes 403a and 404a contacts with a first position in the intraventricular chamber, and the electrode 410a in the pair of stimulation electrodes 410a and 411a contacts with a second position in the intraventricular chamber which is different from the first position. The two pairs of electrodes respectively sense local sense signals generated by changes in membrane potentials of myocardial cells at the first position and the second position. When the local sense signals at the first position and the second position satisfy a preset condition, the pairs of stimulation electrodes respectively apply an electrical stimulation impulse to the first position and the second position to enhance the contractility of the myocardial cells. For example, a control module of the apparatus 400a may determine whether an electrical activity of myocardial cells at the first position and/or the second position corresponds to a depolarizing conduction process of the ventricle according to discharge intensities of myocardial cells and/or a temporal sequence indicated by the local sense signals at the first position and the second position, and if yes, apply the electrical stimulation impulse to myocardial tissue at the first position and/or the second position in the absolute refractory period of the global activation event.

The implantable heart failure treatment apparatus 400b in the embodiment shown in FIG. 3B is substantially the same as the implantable heart failure treatment apparatus 300c of FIG. 2C, except that the apparatus 400b further includes another anchoring member 409b and another stimulation electrode 410b arranged on the anchoring member 409b, where the electrode 404b constituted by a part of the housing 401b forms two stimulation electrode pairs with the electrodes 403b and 410b, respectively. The apparatus 400c shown in FIG. 3C substantially has the same structure as that of the apparatus 300a shown in FIG. 2A, except that the apparatus 400c further includes another anchoring member 409c, and an electrode 404c constituted by a part of the housing 401c forms two stimulation electrode pairs with the electrodes 403c and 410c, respectively. Similar to the description of the apparatus 400a in FIG. 3A, the apparatuses 400b and 400c may also be configured to use two pairs of stimulation electrodes to implement the detection processing of local activation events at different positions in the heart and the delivery of electrical stimulation impulses to the positions, which will not be described in detail herein again.

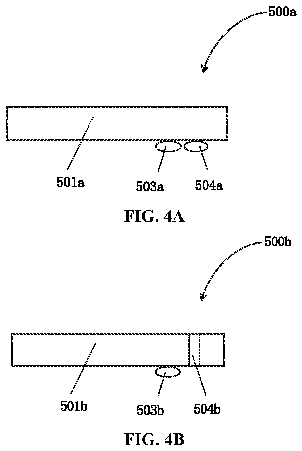

FIG. 4A and FIG. 4B are respectively schematic diagrams of implantable heart failure treatment apparatuses 500a and 500b according to different embodiments of the present application, which schematically show positional arrangements of the pair of stimulation electrodes relative to the housing. As shown in FIG. 4A, the apparatus 500a includes a housing 501a and a pair of stimulation electrodes including electrodes 503a and 504a that attached to the housing 501a. As described above, the apparatus 500a is configured to be arranged in a venous vessel outside the heart of the patient, and the predetermined stimulation position is epicardial myocardial tissue near venous vessels. The pair of stimulation electrodes are configured to detect a local sense (LS) signal generated by discharge of myocardial cells at a specific epicardial position, and to apply an electrical stimulation impulse for enhancing the contractility of myocardial cells in the heart of the patient to the corresponding position. As shown in FIG. 4A, the housing 501a and the pair of stimulation electrodes attached to the surface of the housing are configured to fit to a venous vessel wall on the outer side of the heart to secure the apparatus 500a in the vessel, so that at least one electrode in the pair of stimulation electrodes is arranged abutting against a side of the vessel wall adjacent to the epicardium.

Furthermore, in some embodiments, the apparatus 500a may also have an anchoring member which is a stent, balloon, support arm, or other similar inflatable structure or is an anchor or hook structure, so as to fit to the venous vessel wall to secure the apparatus 500a. In some other embodiments, at least a part of the housing 501a is configured to have such a size that can be fixedly arranged in a relatively narrow segment of the venous vessel on the outer side of the heart, thereby securing the housing 501a in the venous vessel. The implantable heart failure treatment apparatus 500b shown in FIG. 4B is substantially the same as the apparatus 500a in FIG. 4A, except that the configuration of the electrode 504b in the pair of stimulation electrodes is replaced with a part of the housing 501a, e.g., an annular region on the housing 501b which is not covered by an insulating material. In some embodiments, the electrode 504b may also be a partial region of the annular region facing the myocardium, e.g., a semi-annular region facing the myocardium, or a region in the shape of an arc smaller or larger than a semi-annular shape.

The implantable heart failure treatment apparatuses 500a and 500b shown in FIG. 4A and FIG. 4B wirelessly communicate with the subcutaneous implantable cardioverter-defibrillator 200 shown in FIG. 1 to transmit an acquired local sense signal and/or signal sense result to the subcutaneous implantable cardioverter-defibrillator 200. Then, the subcutaneous implantable cardioverter-defibrillator 200 may perform a determination operation based on the far-field sense signal and/or the local sense signal, and send, to the implantable heart failure treatment apparatus 500a or 500b when appropriate, an instruction of delivering an electrical stimulation impulse, where the instruction includes, but is not limited to, a delivery time and an impulse parameter. Then, based on the received instruction, the apparatus 500a or 500b controls the pair of stimulation electrodes to apply the electrical stimulation impulse to the predetermined stimulation position (myocardial adventitia) that the pair of stimulation electrodes contact with or correspond to, to enhance the contractility of myocardium.

It should be noted that the embodiments shown in FIG. 2A to FIG. 4B are for the purpose of schematically illustrating the possible positional arrangements of the pair of stimulation electrodes and the housing. The material, structure, shape, etc. of the pair of stimulation electrodes in the figures may adopt any one or combination of the technical features of the electrodes 103 and 104 described in the embodiment shown in FIG. 1, or may adopt any feasible positional arrangement or configuration that can achieve their functions, which will not be described in detail herein again. Furthermore, for the sake of simplification, the insulating material or structure between the electrodes are not shown in some figures, but it is apparent that there is an insulating structure or material provided between electrodes. For example, the housing may be electrically conductive as a whole, and a partial region of the housing is coated or wrapped by an insulating material to form an electrode. For another example, the housing of the apparatus may be made entirely of an insulating material, and one or more electrodes extend from the housing and are connected to a circuit module in the housing.

It should be noted that, the term "subcutaneous defibrillation assembly" in the present application refers to any feasible configuration that has electrodes and can detect a global activation event of the heart of the patient, generate a far-field sense (or referred to as Global Sense (GS)) signal indicative of the global activation event, and deliver a defibrillation wave to the patient when the patient has malignant arrhythmia. For example, in some embodiments, the "subcutaneous defibrillation assembly" may be a subcutaneous implantable cardioverter-defibrillator (S-ICD). In some other embodiments, the "subcutaneous defibrillation assembly" may have all or part of its components implanted under the skin and other parts (including all components) implanted under muscle. In some other embodiments, the "subcutaneous defibrillation assembly" may be partially or entirely located outside the body, and for example, is a wearable cardioverter-defibrillator (WCD).

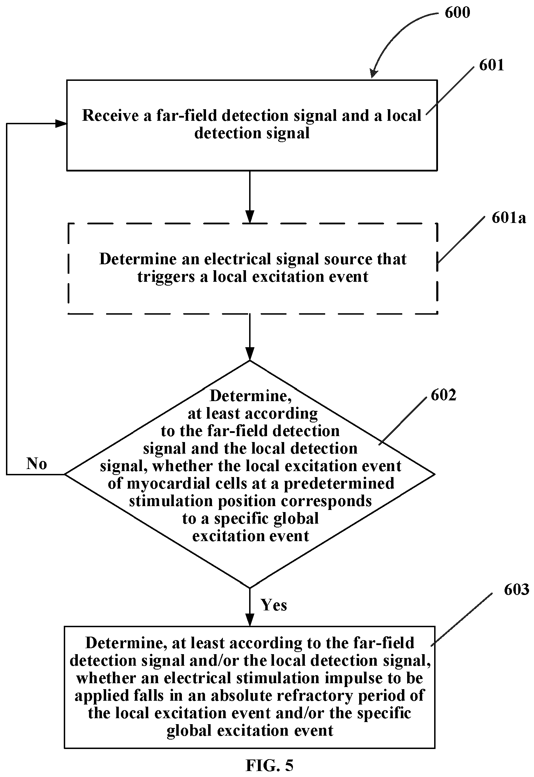

FIG. 5 is a flowchart of a method 600 of controlling a cardiac therapy apparatus according to an embodiment of the present application. The steps of the method will be described below using the cardiac therapy apparatus shown in FIG. 1 as an example.

In step 601, the first control component 206 of the subcutaneous defibrillation assembly 200 receives, from the second control component 106 of the leadless myocardial stimulation assembly 100, a discharge activity of myocardial cells detected by the pair of detection electrodes 103 and 104 at a predetermined stimulation position in the heart, and generates a local sense signal indicative of the discharge activity of the myocardial cells. At the same time, the first control component 206 also acquires a far-field sense signal indicative of a global activation event in the heart of the patient through the pair of detection electrodes 202 and 203 of the subcutaneous defibrillation assembly 200.

In step 602, the first control component 206 determines, at least according to the received far-field sense signal and local sense signal, whether the local activation event of the myocardial cells at the predetermined stimulation position corresponds to a specific global activation event. In some embodiments, step 602 may include: acquiring a specific event time window corresponding to the specific global activation event; and determining that the local activation event corresponds to the specific global activation event (e.g., R-wave) if a time at which the local activation event is sensed falls in the specific event time window.

The specific event time window refers to a time period associated with a specific event of a cardiac cycle that is determined based on the far-field sense signal (or briefly referred to as a GS signal herein) and/or the local sense signal (or briefly referred to as an LS signal herein). For example, a time period associated with a specific event (e.g., R-wave or QRS complex, etc.) of a cardiac cycle may be determined based on the far-field sense signal sensed by the pair of detection electrodes 202 and 203 of the subcutaneous defibrillation assembly 200. In some embodiments, where the implantable heart failure treatment apparatus 100 includes a plurality of pairs of stimulation electrodes, the specific event time window may also be determined based on a plurality of LS signals provided by the pairs of stimulation electrodes at different predetermined stimulation positions.

Specifically, a starting point of the specific event time window may be determined according to the GS signal and/or the LS signal. In some embodiments, the starting point of the specific event time window may be determined based on a time point at which the specific event is sensed in the far-field sense signal (hereinafter briefly referred to as "GS sense time") and/or a time point at which the local sense signal generated by discharge of myocardial cells at the predetermined stimulation position is sensed (hereinafter briefly referred to as "LS sense time").

In some embodiments, the starting point of the specific event time window (represented by GVT-s in the following formula) may be set in the following manner:

When the LS sense time is later than the GS sense time, i.e., when the difference between the LS sense time and the GS sense time (where the difference is represented by GLSD in the following formula, GLSD = LS - GS) is greater than 0, GVT-s is set to be a moment earlier than the GS sense time (represented by GS in the following formula) by a preset time length (represented by A in the following formula). The specific formula is as follows:

When GLSD>0, GVT-s = GS - A.

On the contrary, when the LS sense time is earlier than or equal to the GS sense time, i.e., when the difference GLSD between the LS sense time and the GS sense time is less than or equal to 0, GVT-s may be set to a moment earlier than the LS sense time (represented by LS in the following formula) by the preset time length. The specific formula is as follows:

When GLSD≤0, GVT-s = LS - A. This formula can also be transformed to GVT-s = GS + GLSD - A.

The above setting can be performed in real time in each cardiac cycle.

It should also be noted that, the cardiac therapy apparatus may also determine, in a preset period of the parameter by monitoring the LS signal and the GS signal, a reference time difference B (i.e., GLSD) between the LS sense time and the GS sense time in the case where the LS sense time is later than the GS sense time and a reference time difference C (i.e., GLSD) between the LS sense time and the GS sense time in the case where the LS sense time is earlier than or equal to the GS sense time. In addition, the value of A is also determined in the preset period, or a more suitable value is obtained. In the subsequent running operation, the setting of GVT-s may be determined based on the reference time differences and the LS sense time or the GS sense time. The specific formula is as follows:

When GLSD>0, GVT-s = LS - B - A.
When GLSD≤0, GVT-s = GS + C - A.

It should be noted that, the specific calculation method is merely exemplary, and the starting point of the specific event time window may also be determined by other methods or based on other parameters. In the above embodiment, the preset time lengths A, B, and C may all be adjustable preset values, where A may range from 0 ms to 40 ms, preferably 20 ms. In some embodiments, the preset time lengths B and C may use similar preset values.

The length of the specific event time window may be a preset time length, which is generally related to the specific type of the specific event. If the electrical signal source is an electrical impulse from an atrium, the length of the specific event time window may be 90 ms to 120 ms. If the electrical signal source is an electrical impulse from a ventricle or an external electrical stimulus applied to a ventricle, the length of the specific event time window may be 200 ms to 250 ms. For example, the specific event as an R-wave. For an R-wave caused by a spontaneous impulse of the atrium (e.g., by the sinoatrial node), the length of the specific event time window (or referred to as R-wave time window) may be set to 30 ms to 130 ms, preferably 90 ms to 120 ms, or 90 ms. For an R-wave caused by a spontaneous activity of the ventricle (e.g., premature ventricular contraction) or an external electrical stimulus to the ventricular tissue, the length of the R-wave time window may be set to 30 ms to 250 ms, preferably 200 ms to 250 ms, or 150 ms.

Referring to FIG. 5, when determining that the local activation event corresponds to the specific global activation event, the second control component 106 and/or the first control component 206 performs step 603 of determining, at least according to the far-field sense signal and/or the local sense signal, whether an electrical stimulation impulse to be applied falls in an absolute refractory period of the local activation event and/or the specific global activation event. When determining that the local activation event does not correspond to the specific global activation event, the first control component 206 does not deliver the electrical stimulation impulse. If there are two or more leadless heart failure treatment apparatuses in the heart, the second control component 106 performs the above analysis and processing steps on an LS signal from another leadless heart failure treatment apparatus, until processing of LS signals of all the leadless heart failure treatment apparatuses has been completed; and then continues to perform the above signal analysis step on a subsequent far-field sense signal and local sense signal (corresponding to the next heartbeat). For example, the leadless heart failure treatment apparatuses in FIG. 4A and FIG. 4B may be arranged at the epicardial membrane, e.g., arranged on the left or posterior left epicardium through a coronary vein, and monitor an LS signal through the pair of electrodes 503a and 504a or the pair of electrodes 504b and 504b, and determine whether and when to deliver an electrical stimulation impulse. In some embodiments, cardiac pacing may also be performed through the above electrode pair to achieve biventricular therapy.

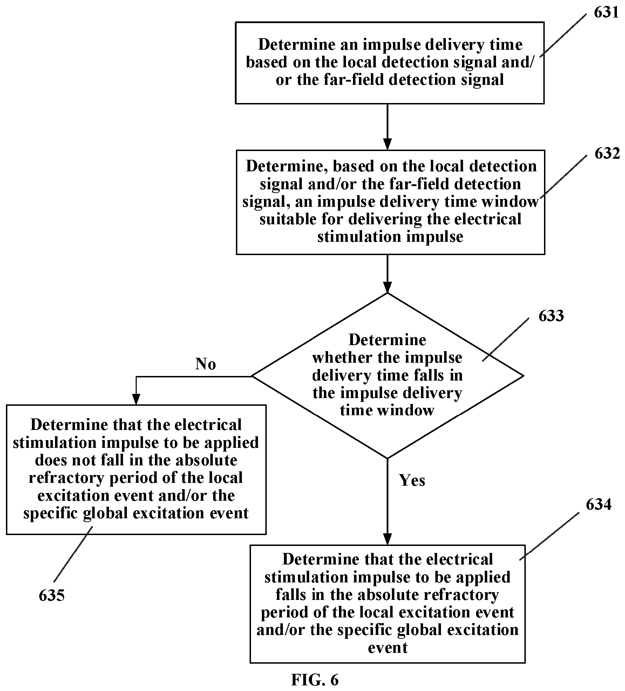

FIG. 6 illustrates specific sub-steps of step 603 of the method 600 shown in FIG. 5 according to some embodiments. The steps of the method will be described below using the cardiac therapy apparatus shown in FIG. 1 as an example.

In step 631, the second control component 106 and/or the first control component 206 determines an impulse delivery time based on the local sense signal and/or the far-field sense signal.

In step 632, the second control component 106 and/or the first control component 206 determines, based on the local sense signal and/or the far-field sense signal, an impulse deliverable time window suitable for delivering the electrical stimulation impulse.

In step 633, the second control component 106 and/or the first control component 206 determines whether the impulse delivery time falls in the impulse deliverable time window.

When the impulse delivery time falls in the impulse deliverable time window, the second control component 106 performs step 634 to determine that the electrical stimulation impulse to be applied falls in the absolute refractory period of the local activation event and/or the specific global activation event. Then, the electrical stimulation impulse for enhancing the contractility of myocardium of the patient may be applied to the predetermined stimulation position through the pair of stimulation electrodes 103 and 104 in subsequent steps.

When the impulse delivery time does not fall in the impulse deliverable time window, the second control component 106 performs step 635 to determine that the electrical stimulation impulse to be applied does not fall in the absolute refractory period of the local activation event and/or the specific global activation event, and therefore does not deliver the electrical stimulation impulse to the predetermined stimulation position.

In the present application, the impulse delivery time (or briefly referred to as CSDT herein) refers to a time point at which the pair of stimulation electrodes deliver the electrical stimulation impulse, which is determined based on the obtained GS signal and/or LS signal. The impulse delivery time may be determined in different manners. The setting of the CSDT ensures that the delivery time of the impulse stimulus is within the absolute refractory period of the local activation event and/or the specific global activation event.

In some embodiments, the impulse delivery time may be determined according to the LS sense time. For example, the impulse delivery time may be a time later than the LS sense time by a preset time length. The specific formula is as follows:

$$CSDT = LS + D.$$

If the electrical activity at the preset position of the myocardium is caused by pacing,

$$CSDT = \text{pacing impulse delivery time} + D$$

In some other embodiments, the impulse delivery time may be determined based on the GS sense time and the GLSD. For example, the impulse delivery time may be a sum of the GS sense time, the GLSD, and the preset time length. The specific formula is as follows:

$$CSDT = GS + GLSD + D.$$

As describe above, a reference time difference E (i.e., GLSD) between the LS sense time and the GS sense time in the case where the LS sense time is later than the GS sense time and a reference time difference F (i.e., GLSD) between the LS sense time and the GS sense time in the case where the LS sense time is earlier than or equal to the GS sense time may also be determined in the preset period by sensing the LS signal and the GS signal. In the subsequent running operation, CSDT may be determined based on the reference time differences and the LS sense time or the GS sense time. The specific formula is as follows:

When GLSD>0, CSDT = GS + E + D.
When GLSD≤0, CSDT = GS + F + D.

The reference time differences E and F may be determined in real time for each cardiac cycle, or may be determined as a preset value in a set period, e.g., by calculating an average, median, or other suitable statistical number of the values of a plurality of (e.g., 16) cardiac cycles.

It should be noted that, the specific calculation method is merely exemplary, and impulse delivery time may also be determined by other methods or based on other parameters. In the above embodiment, the preset time length D may range from 10 ms to 80 ms, preferably 40 ms. If the electrical activity is caused by pacing, the preset time length D may range from 10 ms to 100 ms, preferably 60 ms.

The impulse deliverable time window refers to a time period suitable for delivering the electrical stimulation impulse, during which the delivery of the electrical stimulation impulse to the predetermined stimulation position through the stimulation electrodes will not or hardly cause a re-polarization event of the predetermined stimulation position and other myocardial tissue that the electrical stimulation impulse can affect. The setting of the impulse deliverable time window as a determination condition enables the implantable heart failure treatment apparatus to deliver the electrical stimulation impulse at an appropriate time point in the absolute refractory period of myocardial tissue at the position where the electrical stimulation impulse is applied and myocardial tissue that the electrical stimulation impulse can affect, so as to enhance the contractility of myocardium while avoiding the risk of ventricular tachycardia (VT) or ventricular fibrillation (VF) associated with malignant ventricular arrhythmias.

In some embodiments, the starting point of the impulse deliverable time window may be determined in the same manner as the starting point of the specific event time window, so the details will not be repeated herein. In some embodiments, the starting point of the specific event time window and the starting point of the impulse deliverable time window may also be determined in any other possible manner. For example, when the implantable heart failure treatment apparatus includes a plurality of pairs of stimulation electrodes, e.g., when the apparatus is implanted in both the right ventricle and the left ventricle, the specific event time window and the impulse deliverable time window may also be determined based on a GS signal and an LS signal that is from the pair of stimulation electrodes that earliest sense a local activation event in a cardiac cycle. Of course, the impulse deliverable time window may also be determined based on the LS signal, the GS signal, and other factors mentioned above.

The length of the impulse deliverable time window (or referred to as an R-wave impulse deliverable time window) may be a preset time length of 100 ms to 500 ms, preferably 100 ms to 400 ms or 150 ms to 300 ms. In some embodiments, the preset time length is 200 ms. The condition of the patient, e.g., whether the patient is taking antiarrhythmic drugs, may affect the absolute refractory period of the myocardium (e.g., amiodarone may prolong the absolute refractory period of the myocardium). The R-wave sensing status (GS and/or LS) may also slightly affect the starting point of the time window. Adaptive adjustment needs to be made according to the condition of the patient and the like.

It should be noted that, the starting points corresponding to the impulse deliverable time window and the specific event time window (or briefly referred to as "detection time window" in part of the description herein) may be the same or different. In other words, the impulse deliverable time window and the specific event time window may be set independently, and may not be associated with or dependent on each other. Specifically, the impulse deliverable time window and the specific event time window may be determined based on the far-field sense signal, and may be adaptively adjusted according to the specific status and the specific myocardial position of the sensed specific event (e.g., R-wave) and other factors such as whether the cardiac electrical activity is spontaneous or due to pacing, so as to cover or meet the requirements of most impulse stimulation scenarios. Specific methods of determining the impulse deliverable time window and the specific event time window will be described in detail below with reference to the accompanying drawings and specific embodiments.

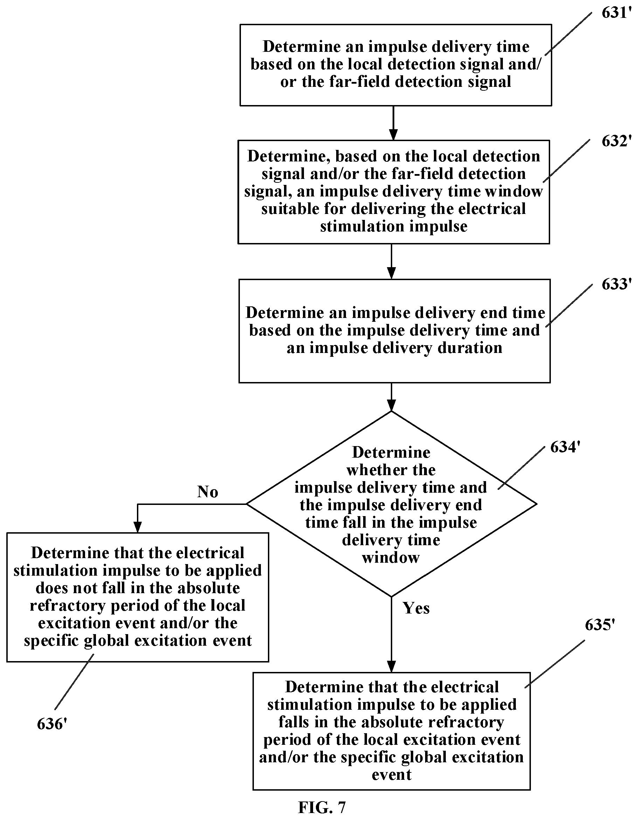

FIG. 7 illustrates specific sub-steps of step 603 of the method 600 shown in FIG. 5 according to some other embodiments. The steps of the method will be described below using the cardiac therapy apparatus shown in FIG. 1 as an example.

Steps 631' and 632' shown in FIG. 7 are the same as steps 631 and 632 shown in FIG. 6, and will not be described in detail herein again. In step 633', the first control component 206 and/or the second control component 106 determines an impulse delivery end time based on the impulse delivery time and an impulse delivery duration.

Then, in step 634', the second control component 106 and/or the first control component 206 determines whether the impulse delivery time and the impulse delivery end time fall in the impulse deliverable time window.

When the impulse delivery time and the impulse delivery end time fall in the impulse deliverable time window, the second control component 106 performs step 635 to determine that the electrical stimulation impulse to be applied falls in the absolute refractory period of the local activation event and/or the specific global activation event. Then, the electrical stimulation impulse for enhancing the contractility of myocardium of the patient is applied to the predetermined stimulation position through the pair of stimulation electrodes 103 and 104 in subsequent steps.

When either of the impulse delivery time and the impulse delivery end time does not fall in the impulse deliverable time window, the second control component 106 performs step 636 to determine that the electrical stimulation impulse to be applied does not fall in the absolute refractory period of the local activation event and/or the specific global activation event, and therefore does not deliver the electrical stimulation impulse.

The specific method of determining the impulse delivery time CSDT has been described in detail above. After the impulse delivery time is determined, assuming that the length of the impulse delivery time is G, the impulse delivery end time (or briefly referred to as CSFT herein) can be determined accordingly:

$$CSFT = CSDT + G.$$

G is obtained according to preset impulse parameters (e.g., stimulation impulse width and impulse number). The parameters may be adjusted and selected according to different heart failure treatment requirements or the specific predetermined stimulation position, and whether the impulse delivery end time falls within the impulse delivery time window.

Optionally, as shown in FIG. 5, the method 600 may further include step 601a prior to step 602. In step 601a, the first control component 206 determines an electrical signal source that triggers a local activation event. The electrical signal source may include a spontaneous electrical impulse from an atrium, a spontaneous electrical impulse from a ventricle, or a ventricular electrical activity caused by an external electrical stimulus applied to a ventricle. In some embodiments, the second control component 106 may determine the electrical signal source, and send the determined electrical signal source to the first control component 206 together with the local sense signal. In some other embodiments, the specific type of the electrical signal source may be determined according to the far-field sense signal. After step 601a, parameters such as the specific event time window and the impulse delivery time may be adjusted in steps 602 and 603 according to the electrical signal source determined in step 601a. For the specific adjustment method, reference can be made to the above description of the methods of determining the specific event time window and the impulse delivery time, etc. For the case where the electrical signal source is an electrical impulse (e.g., a pacing impulse) from outside the heart, reference can be made to the following description of a method of controlling a cardiac therapy apparatus having a pacing function.

In some embodiments, the cardiac therapy apparatus 10 can also provide pacing therapy. Specifically, the stimulation electrodes 103 and 104 of the leadless heart failure treatment apparatus 100 shown in FIG. 1 may be further configured to apply a pacing electrical impulse for adjusting the heart rate of the patient. In some embodiments, the first control component 206 and/or the second control component 106 is further configured to receive a mode selection signal and control the cardiac therapy apparatus to enter a pacing mode or a non-pacing mode according to the mode selection signal. In the non-pacing mode, the first control component 206 performs corresponding operations in accordance with the method 600 shown in FIG. 5, and the details will not be described in detail herein again.

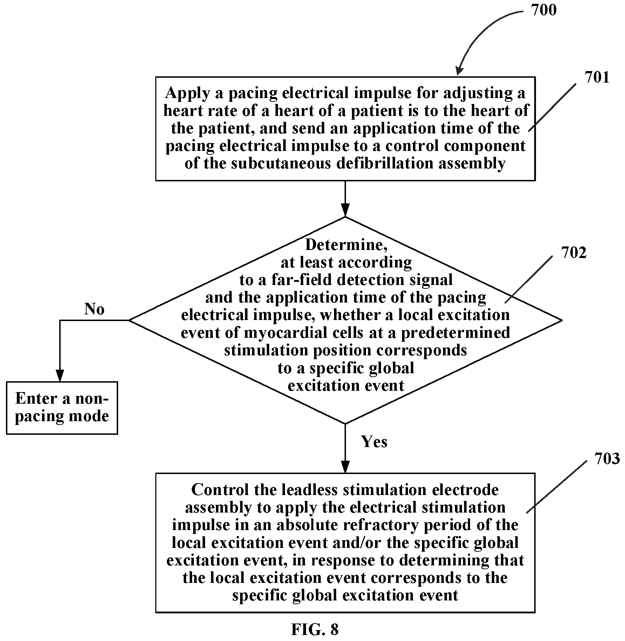

FIG. 8 illustrates a method 700 of controlling the cardiac therapy apparatus 10 in the pacing mode. The steps of the method will be described below using the cardiac therapy apparatus shown in FIG. 1 as an example. Specifically, in the pacing mode, in step 701, the second control component 106 controls the leadless heart failure treatment apparatus 100 to apply a pacing electrical impulse for adjusting the heart rate of the heart of the patient to the patient through the stimulation electrodes 103 and 104, and at the same time, sends pacing electrical impulse information to the first control component 206, where the pacing electrical impulse information includes an application time of the pacing electrical impulse.

Then, in step 702, the first control component 206 determines, at least according to a far-field sense signal and the application time of the pacing electrical impulse, whether a local activation event of myocardial cells at a predetermined stimulation position corresponds to a specific global activation event. For the cardiac therapy apparatus in the pacing mode, the above step is substantially equivalent to a process of the first control component 206 determining whether the pacing impulse achieves capture of the heart after obtaining a signal indicating the application time of the pacing impulse. In some embodiments, when it is determined that the pacing impulse achieves capture of the heart, the application time of the pacing electrical impulse or a sum of the application time of the pacing electrical impulse and a response delay of the myocardium to the stimulation may be used as an beginning time of the local activation event of the myocardial cells at the predetermined stimulation position, and the starting points of the impulse deliverable time window and the specific event time window.

When determining that the local activation event corresponds to the specific global activation event (i.e., determining that the pacing impulse achieves capture of the heart), the second control component 106 and/or the first control component 206 performs step 703 of delivering the electrical stimulation impulse to the pair of stimulation electrodes in an absolute refractory period of the local activation event and/or the specific global activation event. When determining that the local activation event does not correspond to the specific global activation event (i.e., determining that the pacing impulse does not achieve capture of the heart), the first control component 206 does not deliver the electrical stimulation impulse, but instead, performs the corresponding step in FIG. 5, i.e., according to a spontaneous electrical activity mode (entering the non-pacing mode). As described above, parameters such as CSDT in the subsequent step 703 may be adaptively adjusted according to the electrical signal source (i.e., pacing electrical impulse) of the local activation event.

In some embodiments, the first control component 206 is further configured to determine, according to the far-field sense signal, an beginning time of the specific global activation event or a time at which the specific global activation event is sensed, and send a signal indicative of the beginning time of the specific global activation event or the time at which the specific global activation event is sensed to the second control component 106. In this case, the second control component 106 is further configured to determine a local sense signal falling in a time interval before and after the beginning time of the specific global activation event or the time at which the specific global activation event is sensed. Only when there is a local sense signal in the above time range, the second control component 106 sends the local sense signal to the first control component 206, for the first control component 206 to perform further analysis based on the far-field sense signal and the local sense signal. Such an arrangement can effectively reduce the frequency of active communication and signal transmission of the second control component 106, thereby reducing energy consumption of the second control component 106 and prolonging the service life of the second control component 106.

On the contrary, in some other embodiments, the second control component 106 may be further configured to send a local sense signal indicative of the local activation event to the first control component 206, where the local sense signal includes a moment at which the local activation event is sensed. Correspondingly, the first control component 206 may be further configured to determine, based on the received local sense signal, whether there is a far-field sense signal falling in a time interval before and after the time at which the local activation event is sensed. Upon sensing the corresponding far-field sense signal, the first control component 206 performs the above step of determining whether the local activation event corresponds to a specific global activation event. In some embodiments, the first control component 206 is further configured to determine, at least according to the far-field sense signal and the local sense signal, whether to use the subcutaneous defibrillation assembly to deliver a defibrillation wave for treating malignant arrhythmia to the patient. Specifically, when determining according to the far-field sense signal that a defibrillation wave for treating malignant arrhythmia needs to be delivered to the patient, the first control component 206 may further check whether the determination result is correct according to the received local sense signal, and only when checking that the determination result is correct, deliver the defibrillation wave for treating malignant arrhythmia. This can effectively avoid erroneous determination and therefore protect the patient from being injured by an unnecessary electric shock. In some embodiments, when determining according to the far-field sense signal that a defibrillation wave for treating malignant arrhythmia needs to be delivered to the patient, the first control component 206 may send the determination result to the second control component 106, for the second control component 106 to perform the above check process.

In some other embodiments, the second control component 106 may be further configured to determine, according to the sensed local sense signal, an beginning time of the local activation event or a time at which the local activation event is sensed, and send a signal indicative of the beginning time of the local activation event or the time at which the local activation event is sensed to the first control component 206. The first control component 206 is further configured to acquire a far-field sense signal falling in a time interval before and after the beginning time of the local activation event or the time at which the local activation event is sensed. During ECG signal sensing in practice, the time at which the GS signal is sensed may lag behind the real time point due to a delay in ECG signal capturing, or the time at which the LS signal is sensed may be slightly earlier than the time at which the GS signal is sensed due to other reasons. Using the time at which the LS signal is sensed as the starting time of the specific event time window can better cope with this problem, to more accurately determine the correspondence between the local activation event and the global activation event. In this case, after the specific event time window is determined by using the time at which the LS signal is sensed as the starting time of the specific event time window, it is further determined whether the time at which the subsequent GS signal indicative of the global activation event is sensed falls in the specific event time window. When the time at which the GS signal is sensed falls in the specific event time window, it is determined that the local activation even indicated by the LS signal corresponds to the global activation event indicated by the GS signal.

In some embodiments, the first control component 206 is configured to send the far-field sense signal to the second control component 106, and after receiving the far-field sense signal, the second control component 106 determines, at least according to the far-field sense signal and the local sense signal, whether to use the subcutaneous defibrillation assembly to deliver a defibrillation wave for treating malignant arrhythmia to the patient. In some other embodiments, the first control component 206 is configured to directly determine, at least according to the far-field sense signal and the local sense signal, whether to use the subcutaneous defibrillation assembly to deliver a defibrillation wave for treating malignant arrhythmia to the patient.

Since the subcutaneous defibrillation assembly generally uses a large current to deliver the defibrillation wave for treating malignant arrhythmia to the patient, this current may have an unintended impact on the operation of the leadless myocardial stimulation assembly 100. In some embodiments, the first control component 206 is further configured to send to the second control component 106 of the leadless myocardial stimulation assembly 100, a signal indicating that a defibrillation wave for treating malignant arrhythmia is to be delivered, in response to determining to use the subcutaneous defibrillation assembly to deliver the defibrillation wave for treating malignant arrhythmia to the patient. The second control component 106 is further configured to switch the leadless myocardial stimulation assembly 100 to a shock-resistant mode in response to receiving the signal indicating that the defibrillation wave for treating malignant arrhythmia is to be delivered, e.g., to disrupt electrical coupling of the impulse generation module or a sense element in the control component and the stimulation electrodes.

It should be noted that, in some embodiments, the step of "determining whether the electrical stimulation impulse to be applied falls in the absolute refractory period of the local activation event and/or the global activation event" herein includes a case considering only the absolute refractory period of the specific global activation event or considering only the absolute refractory period of the local activation event. In some other embodiments, the above step may also be further defined to consider both the absolute refractory period of the local activation event and the absolute refractory period of the ventricular muscle as a whole. In some embodiments, when the cardiac therapy apparatus includes a plurality of pairs of stimulation electrodes and can acquire a plurality of LS signals, the first control component 206 may also adaptively adjust the specific steps and parameters of the method 600 in consideration of factors such as the positional relationship between the predetermined stimulation position and other local positions. The term "subcutaneous" as used herein is to be interpreted more broadly and should be construed as encompassing situations such as being arranged below a muscle and being partially arranged below the epidermis or muscle.

It should be noted that although several components or modules of the cardiac therapy apparatus, the subcutaneous defibrillation assembly (subcutaneous implantable cardioverter-defibrillator), and the leadless myocardial stimulation assembly (leadless heart failure treatment apparatus) have been mentioned in the above detailed description, such division is merely exemplary and not mandatory. Indeed, according to the embodiments of the present invention, the features and functions of two or more modules described above may be embodied in one module. Similarly, the features and functions of one module described above may be further divided and embodied in a plurality of modules.

Other changes to the disclosed embodiments can be understood and implemented by those of ordinary skill in the art by studying the specification, disclosure, drawings, and appended claims. In the claims, the terms such as "comprise" and any other variant thereof does not exclude other elements and steps, and the terms such as "a", "an", and "the" do not exclude the plural. In the practical application of the present application, one part may perform the functions of a plurality of technical features described in the claims. Any reference signs in the claims should not be construed as limiting the scope.

**Claims**

1.  A cardiac therapy apparatus, comprising:

    a subcutaneous defibrillation assembly, configured to be implanted beneath the skin of a patient and comprising a first control component and a plurality of subcutaneous electrodes, wherein the first control component is configured to sense a global activation event of the heart of the patient through at least a part of the plurality of subcutaneous electrodes, generate a far-field sense signal indicative of the global activation event, and deliver a defibrillation wave to the patient through at least a part of the plurality of subcutaneous electrodes when the patient has malignant arrhythmia; and
    a leadless myocardial stimulation assembly, configured to contact a predetermined stimulation position of the heart of the patient and comprising a second control component and a plurality of stimulation assembly electrodes, wherein the second control component is configured to sense a local activation event of myocardium at the predetermined stimulation position through at least a part of the plurality of stimulation assembly electrodes, generate a local sense signal indicative of the local activation event, and apply to the predetermined stimulation position an electrical stimulation impulse for enhancing the contractility of myocardial cells in the heart of the patient through at least a part of the plurality of stimulation assembly electrodes,
    wherein the first control component is coupled to the second control component through wireless communication, and the first control component and/or the second control component are/is configured to determine, at least according to the far-field sense signal and the local sense signal, whether to apply the electrical stimulation impulse to the predetermined stimulation position.

2.  The cardiac therapy apparatus according to claim 1, wherein the determining, according to the far-field sense signal and the local sense signal, whether to apply the electrical stimulation impulse to the predetermined stimulation position comprises determining whether to apply the electrical stimulation impulse to the predetermined stimulation position based on a determination result of at least one of the following steps:

    determining, at least according to the far-field sense signal and the local sense signal, whether the local activation event of myocardial cells at the predetermined stimulation position corresponds to a specific global activation event; and
    determining, at least according to the far-field sense signal and/or the local sense signal, whether the electrical stimulation impulse to be applied falls in an absolute refractory period of the local activation event and/or a specific global activation event.

3.  The cardiac therapy apparatus according to claim 1, wherein the determining, at least according to the far-field sense signal and the local sense signal, whether to apply the electrical stimulation impulse to the predetermined stimulation position comprises: determining to apply the electrical stimulation impulse to the predetermined stimulation position, in response to determining that the local activation event corresponds to a specific global activation event and/or in response to determining that the electrical stimulation impulse to be applied falls in an absolute refractory period of the local activation event and/or the specific global activation event.

4. The cardiac therapy apparatus according to claim 1, wherein the leadless myocardial stimulation assembly comprises:

a housing, having attached thereto an anchoring member for securing the housing to the heart of the patient; and
an impulse generation module, received within the housing, and configured to generate the electrical stimulation impulse under control of the second control component; and
the plurality of stimulation assembly electrodes comprise a pair of stimulation electrodes coupled to the housing, the impulse generation module is electrically coupled to the pair of stimulation electrodes, the pair of stimulation electrodes are configured to contact the predetermined stimulation position of the heart of the patient, and the second control component is configured to sense the local activation event of the myocardium at the predetermined stimulation position through the pair of stimulation electrodes, generate the local sense signal indicative of the local activation event, and apply the electrical stimulation impulse to the predetermined stimulation position through the pair of stimulation electrodes.

5. The cardiac therapy apparatus according to claim 4, wherein the housing of the leadless myocardial stimulation assembly is configured to be received within a venous vessel on a external surface of the heart of the patient, and the predetermined stimulation position comprises an epicardium.

6. The cardiac therapy apparatus according to claim 1, wherein the local sense signal comprises a time at which the local activation event is sensed.

7. The cardiac therapy apparatus according to claim 2, wherein the determining, at least according to the far-field sense signal and the local sense signal, whether the local activation event of myocardial cells at the predetermined stimulation position corresponds to a specific global activation event comprises:

acquiring a specific event time window corresponding to the specific global activation event; and determining that the local activation event corresponds to the specific global activation event if a time at which the local activation event is sensed falls in the specific event time window; or
acquiring a specific event time window corresponding to the local activation event; and determining that the local activation event corresponds to the specific global activation event if a time at which the specific global activation event is sensed falls in the specific event time window.

8. The cardiac therapy apparatus according to claim 2, wherein the determining, at least according to the far-field sense signal and/or the local sense signal, whether the electrical stimulation impulse to be applied falls in an absolute refractory period of the local activation event and/or a specific global activation event comprises:

determining an impulse delivery time of the electrical stimulation impulse based on the local sense signal and/or the far-field sense signal;
determining, based on the local sense signal and/or the far-field sense signal, an impulse deliverable time window suitable for delivering the electrical stimulation impulse; and
determining that the electrical stimulation impulse to be applied falls in the absolute refractory period of the local activation event and/or the specific global activation event, when the impulse delivery time falls in the impulse deliverable time window.

9. The cardiac therapy apparatus according to claim 2, wherein the determining, at least according to the far-field sense signal and/or the local sense signal, whether the electrical stimulation impulse to be applied falls in an absolute refractory period of the local activation event and/or a specific global activation event comprises:

determining an impulse delivery time of the electrical stimulation impulse based on the local sense signal and/or the far-field sense signal;
determining, based on the local sense signal and/or the far-field sense signal, an impulse deliverable time window suitable for delivering the electrical stimulation impulse;
determining an impulse delivery end time based on the impulse delivery time and an impulse delivery duration; and
determining that the electrical stimulation impulse to be applied falls in the absolute refractory period of the local activation event and/or the specific global activation event, when the impulse delivery time and the impulse delivery end time fall in the impulse deliverable time window.

10. The cardiac therapy apparatus according to claim 8 or 9, wherein the determining, based on the local sense signal and/or the far-field sense signal, an impulse deliverable time window suitable for delivering the electrical stimulation impulse comprises:

determining a beginning time of the deliverable impulse based on the local sense signal and/or the far-field sense signal; and
determining the impulse deliverable time window according to the beginning time of the deliverable impulse and a predetermined time length.

11. The cardiac therapy apparatus according to claim 10, wherein the predetermined time length ranges from 100 ms to 500 ms or from 150 ms to 300 ms.

12. The cardiac therapy apparatus according to claim 9, wherein the first control component and the second control component are configured to adjust an impulse delivery parameter when the impulse delivery end time does not fall in the impulse deliverable time window, so that the adjusted impulse delivery end time falls in the impulse deliverable time window, and apply the adjusted electrical stimulation impulse to the predetermined stimulation position.

13. The cardiac therapy apparatus according to claim 1, wherein

the first control component is further configured to determine, according to the far-field sense signal, an beginning time of the specific global activation event or a time at which the specific global activation event is sensed, and send a signal indicative of the beginning time of the specific global activation event or the time at which the specific global activation event is sensed to the second control component; and the second control component is further configured to acquire a local sense signal falling in a time interval before and after the beginning time of the specific global activation event or the time at which the specific global activation event is sensed, and transmit the local sense signal to the first control component; or
the second control component is further configured to determine, according to the local sense signal, an beginning time of the local activation event or a time at which the local activation event is sensed, and send a signal indicative of the beginning time of the local activation event or the time at which the local activation event is sensed to the first control component; and the first control component is further configured to acquire a far-field sense signal falling in a time interval before and after the beginning time of the local activation event or the time at which the local activation event is sensed, and transmit the far-field sense signal to the second control component.

14. The cardiac therapy apparatus according to claim 1, wherein the second control component is further configured to send the local sense signal to the first control component, or the first control component is further configured to send the far-field sense signal to the second control component.

15. The cardiac therapy apparatus according to claim 2, wherein the specific global activation event corresponds to an R-wave or QRS complex in a far-field electrocardiogram, and the local sense signal corresponds to an R-wave in a near-field electrocardiogram.

16. The cardiac therapy apparatus according to claim 1, wherein the first control component and/or the second control component are/is further configured to determine, at least according to the far-field sense signal and the local sense signal, whether to deliver a defibrillation wave for treating malignant arrhythmia to the patient via the subcutaneous defibrillation assembly.

17. The cardiac therapy apparatus according to claim 1, wherein the first control component is further configured to send, to the second control component, a signal indicating that a defibrillation wave for treating malignant arrhythmia is to be delivered, in response to determining to deliver the defibrillation wave for treating malignant arrhythmia to the patient via the subcutaneous defibrillation assembly.

18. The cardiac therapy apparatus according to claim 17, wherein the second control component is further configured to switch the leadless myocardial stimulation assembly to a shock-resistant mode in response to receiving the signal indicating that the defibrillation wave for treating malignant arrhythmia is to be delivered.

19. The cardiac therapy apparatus according to claim 2, wherein the first control component is further configured to determine an electrical signal source that triggers the global activation event, and determine whether the local activation event of myocardial cells at the predetermined stimulation position corresponds to a specific global activation event at least according to the electrical signal source, the local sense signal, and the far-field sense signal.

20. The cardiac therapy apparatus according to claim 19, wherein the determining whether the local activation event of myocardial cells at the predetermined stimulation position corresponds to a specific global activation event at least according to the electrical signal source, the local sense signal, and the far-field sense signal comprises:
determining a length of a specific event time window corresponding to the specific global activation event according to the electrical signal source, wherein the length of the specific event time window is 90 ms to 120 ms if the electrical signal source is an electrical impulse from an atrium, and the length of the specific event time window is 200 ms to 250 ms if the electrical signal source is an electrical impulse from a ventricle or an external electrical stimulus applied to a ventricle.

21. The cardiac therapy apparatus according to claim 19, wherein the determining, at least according to the far-field sense signal and/or the local sense signal, whether the electrical stimulation impulse to be applied falls in an absolute refractory period of the local activation event and/or a specific global activation event comprises:

determining the impulse delivery time based on the electrical signal source in combination with the local sense signal and/or the far-field sense signal;
determining, based on the electrical signal source in combination with the local sense signal and/or the far-field sense signal, an impulse deliverable time window suitable for delivering the electrical stimulation impulse; and
delivering the electrical stimulation impulse to a pair of stimulation electrodes when the impulse delivery time falls in the impulse deliverable time window.

22. The cardiac therapy apparatus according to claim 19, wherein the electrical signal source that triggers the local activation events comprises an electrical impulse from an atrium, an electrical impulse from a ventricle, or an external electrical stimulus applied to a ventricle.

23. The cardiac therapy apparatus according to claim 2, wherein the second control component is further configured to control the leadless electrode assembly to selectively apply a pacing electrical impulse for adjusting the heart rate of the patient to the predetermined stimulation position, and send an application time of the pacing electrical impulse to the first control component, and the first control component is further configured to determine whether the local activation event of myocardial cells at the predetermined stimulation position corresponds to the specific global activation event at least according to the far-field sense signal and the application time of the pacing electrical impulse.

24. The cardiac therapy apparatus according to claim 23, wherein the first control component or the second control component is further configured to receive a mode selection signal and control the cardiac therapy apparatus to enter a pacing mode or a non-pacing mode according to the mode selection signal, wherein in the non-pacing mode, the first control component determines whether the local activation event of myocardial cells at the predetermined stimulation position corresponds to the specific global activation event at least according to the far-field sense signal and the local sense signal, and in the pacing mode, the first control component determines whether the local activation event of myocardial cells at the predetermined stimulation position corresponds to the specific global activation event at least according to the far-field sense signal and the application time of the pacing electrical impulse.

25. The cardiac therapy apparatus according to claim 24, wherein in the pacing mode, the first control component is configured to determine capture of the heart of the patient based on the far-field sense signal and determine an beginning point of the local activation event of myocardial cells at the predetermined stimulation position according to the application time of the pacing electrical impulse after the capture, and the first control component and/or the second control component are/is configured to determine whether to apply the electrical stimulation impulse to the predetermined stimulation position at least according to the field sense signal and the beginning point of the local activation event.

26. A cardiac therapy apparatus, comprising:

a subcutaneous defibrillation assembly, configured to be implanted beneath the skin of a patient and comprising a first control component and a plurality of subcutaneous electrodes, wherein the first control component is configured to sense a global activation event of the heart of the patient through at least a part of the plurality of subcutaneous electrodes, generate a far-field sense signal indicative of the global activation event, and deliver a defibrillation wave to the patient through at least a part of the plurality of subcutaneous electrodes when the patient has malignant arrhythmia; and
a leadless myocardial stimulation assembly, configured to contact a predetermined stimulation position of the heart of the patient and comprising a second control component and a plurality of stimulation assembly electrodes, wherein the second control component is configured to apply, to the predetermined stimulation position through at least a part of the plurality of stimulation assembly electrodes, an electrical stimulation impulse for enhancing the contractility of myocardial cells in the heart of the patient, and the second control component is configured to apply, to the predetermined stimulation position through at least a part of the plurality of stimulation assembly electrodes, a pacing electrical impulse for adjusting the heart rate of the patient, and generate a pacing electrical impulse signal indicating the pacing electrical impulse applied; and

wherein the first control component is coupled to the second control component through wireless communication, and the first control component is configured to determine, at least according to the far-field sense signal and the pacing electrical impulse signal, whether to apply the electrical stimulation impulse to the predetermined stimulation position.

27. The cardiac therapy apparatus according to claim 26, wherein after the second control component is configured to apply, to the predetermined stimulation position through at least a part of the plurality of stimulation assembly electrodes, a pacing electrical impulse for adjusting the heart rate of the patient, the first control component is further configured to determine capture of the heart of the patient and determine a beginning point of a local activation event of myocardial cells at the predetermined stimulation position according to an application time of the pacing electrical impulse based on the capture; and the first control component and/or the second control component are/is configured to determine whether to apply the electrical stimulation impulse to the predetermined stimulation position at least according to the far-field sense signal and the beginning point of the local activation event.

28. A subcutaneous implantable cardioverter-defibrillator, comprising:

a housing, configured to be implanted under the skin of a patient;
a plurality of electrodes, coupled to the housing and configured to be implanted under the skin of the patient;
a control module, coupled to a leadless heart failure treatment apparatus in contact with a predetermined stimulation position of the heart of the patient by wireless communication, to receive a local sense signal indicative of a local activation event of the myocardium at the predetermined stimulation position and generated by the leadless heart failure treatment apparatus based on sensation of the local activation event, the control module being configured to sense a global activation event of the heart of the patient through at least a part of the plurality of electrodes, generate a far-field sense signal indicative of the global activation event, and determine, at least according to the -far-field sense signal and the local sense signal, whether the local activation event of myocardial cells at the predetermined stimulation position corresponds to a specific global activation event or determine, at least according to the far-field sense signal and/or the local sense signal, whether an electrical stimulation impulse to be applied by the leadless heart failure treatment apparatus falls in an absolute refractory period of the local activation event and/or a specific global activation event, to determine whether to use the leadless heart failure treatment apparatus to apply the electrical stimulation impulse to the predetermined stimulation position; and
an impulse generation module, received within the housing, electrically coupled to at least a part of the plurality of electrodes, and configured to deliver a defibrillation wave for treating malignant arrhythmia to the patient through at least a part of the plurality of electrodes under control of the control module.

29. The subcutaneous implantable cardioverter-defibrillator according to claim 28, wherein the determining, at least according to the far-field sense signal and the local sense signal, whether the local activation event of myocardial cells at the predetermined stimulation position corresponds to a specific global activation event comprises:

acquiring a specific event time window corresponding to the specific global activation event; and determining that the local activation event corresponds to the specific global activation event if a time at which the local activation event is sensed falls in the specific event time window; or
acquiring a specific event time window corresponding to the local activation event; and determining that the local activation event corresponds to the specific global activation event if a time at which the specific global activation event is sensed falls in the specific event time window.

30. The subcutaneous implantable cardioverter-defibrillator according to claim 28, wherein the control module and/or the leadless heart failure treatment apparatus are/is further configured to determine, at least according to the far-field sense signal and/or the local sense signal, whether the electrical stimulation impulse to be applied falls in an absolute refractory period of the local activation event and/or the specific global activation event, in response to determining that the local activation event corresponds to the specific global activation event.

31. The subcutaneous implantable cardioverter-defibrillator according to claim 30, wherein the determining, at least according to the far-field sense signal and/or the local sense signal, whether the electrical stimulation impulse to be applied falls in an absolute refractory period of the local activation event and/or a specific global activation event comprises:

determining an impulse delivery time of the electrical stimulation impulse based on the local sense signal and/or the far-field sense signal; determining, based on the local sense signal and/or the far-field sense signal, an impulse deliverable time window suitable for delivering the electrical stimulation impulse; and determining that the electrical stimulation impulse to be applied falls in the absolute refractory period of the local activation event and/or the specific global activation event, when the impulse delivery time falls in the impulse deliverable time window.

32. The subcutaneous implantable cardioverter-defibrillator according to claim 30, wherein the determining, at least according to the far-field sense signal and/or the local sense signal, whether the electrical stimulation impulse to be applied falls in an absolute refractory period of the local activation event and/or a specific global activation event comprises:

determining an impulse delivery time of the electrical stimulation impulse based on the local sense signal and/or the far-field sense signal; determining, based on the local sense signal and/or the far-field sense signal, an impulse deliverable time window suitable for delivering the electrical stimulation impulse; determining an impulse delivery end time based on the impulse delivery time and an impulse delivery interval; and determining that the electrical stimulation impulse to be applied falls in the absolute refractory period of the local activation event and/or the specific global activation event, when the impulse delivery time and the impulse delivery end time fall in the impulse deliverable time window.

33. The subcutaneous implantable cardioverter-defibrillator according to claim 31 or 32, wherein the determining, based on the local sense signal and/or the far-field sense signal, an impulse deliverable time window suitable for delivering the electrical stimulation impulse comprises:

determining a beginning time of the deliverable impulse based on the local sense signal and/or the far-field sense signal; and determining the impulse deliverable time window according to the beginning time of the deliverable impulse and a predetermined time length.

34. The subcutaneous implantable cardioverter-defibrillator according to claim 28, wherein the control module is further configured to determine, according to the far-field sense signal, an beginning time of the specific global activation event or a time at which the specific global activation event is sensed, and send a signal indicative of the beginning time of the specific global activation event or the time at which the specific global activation event is sensed to the leadless heart failure treatment apparatus.

35. A method of controlling a cardiac therapy apparatus, wherein the cardiac therapy apparatus comprises a subcutaneous defibrillation assembly and a leadless myocardial stimulation assembly, the subcutaneous defibrillation assembly is configured to be implanted under the skin of a patient and comprises a first control component and a plurality of subcutaneous electrodes, and the leadless myocardial stimulation assembly is configured to contact a predetermined stimulation position of the heart of the patient and comprises a plurality of stimulation assembly electrodes and a second control component coupled to the first control component by wireless communication, the method comprising:

sensing, by the first control component, a global activation event of the heart of the patient through at least a part of the plurality of subcutaneous electrodes, and generating a far-field sense signal indicative of the global activation event; sensing, by the second control component, a local activation event of myocardium at the predetermined stimulation position through at least a part of the plurality of stimulation assembly electrodes, and generating a local sense signal indicative of the local activation event; and determining, by the first control component and/or the second control component at least according to the far-field sense signal and the local sense signal, whether to apply the electrical stimulation impulse to the predetermined stimulation position.

36. A non-volatile computer-readable storage medium, having a computer program stored thereon, wherein the computer program, when executed by a processor, causes the processor to implement the steps of the method according to claim 35.

**FIG. 1**

300a

302a

301a          304a    303a

**FIG. 2A**

300b

302b

301b          304b    303b

**FIG. 2B**

300c

302c

301c          304c    303c

**FIG. 2C**

400a

411a  409a  410a

401a  404a  402a  403a

**FIG. 3A**

400b

404b (411b)

409b  410b

401b  402b  403b

**FIG. 3B**

400c

404c
(411c)  402c

401c  409c  410c  403c

**FIG. 3C**

500a

501a          503a      504a

**FIG. 4A**

500b

501b          503b      504b

**FIG. 4B**

600

601

Receive a far-field detection signal and a local detection signal

601a

Determine an electrical signal source that triggers a local excitation event

602

No

Determine, at least according to the far-field detection signal and the local detection signal, whether the local excitation event of myocardial cells at a predetermined stimulation position corresponds to a specific global excitation event

Yes

603

Determine, at least according to the far-field detection signal and/or the local detection signal, whether an electrical stimulation impulse to be applied falls in an absolute refractory period of the local excitation event and/or the specific global excitation event

FIG. 5

Determine an impulse delivery time
based on the local detection signal and/
or the far-field detection signal

631

Determine, based on the local detection
signal and/or the far-field detection
signal, an impulse delivery time window
suitable for delivering the electrical
stimulation impulse

632

Determine
whether the impulse
delivery time falls in
the impulse delivery
time window

633

No

Determine that the electrical
stimulation impulse to be applied
does not fall in the absolute
refractory period of the local
excitation event and/or the
specific global excitation event

635

Yes

Determine that the electrical
stimulation impulse to be applied
falls in the absolute refractory
period of the local excitation event
and/or the specific global excitation
event

634

FIG. 6

Determine an impulse delivery time
based on the local detection signal and/
or the far-field detection signal
631'

Determine, based on the local detection
signal and/or the far-field detection
signal, an impulse delivery time window
suitable for delivering the electrical
stimulation impulse
632'

Determine an impulse delivery end time
based on the impulse delivery time and
an impulse delivery duration
633'

Determine
whether the
impulse delivery time and
the impulse delivery end
time fall in the impulse
delivery time
window
634'

No

Yes

Determine that the electrical
stimulation impulse to be applied
does not fall in the absolute
refractory period of the local
excitation event and/or the
specific global excitation event

636'

635'

Determine that the electrical
stimulation impulse to be applied
falls in the absolute refractory
period of the local excitation event
and/or the specific global excitation
event

FIG. 7

700

701

Apply a pacing electrical impulse for adjusting a
heart rate of a heart of a patient is to the heart of
the patient, and send an application time of the
pacing electrical impulse to a control component
of the subcutaneous defibrillation assembly

702

Determine,
at least according
to a far-field detection signal
and the application time of the pacing
electrical impulse, whether a local excitation
event of myocardial cells at a predetermined
stimulation position corresponds
to a specific global
excitation event

No

Enter a non-
pacing mode

Yes

703

Control the leadless stimulation electrode
assembly to apply the electrical stimulation
impulse in an absolute refractory period of the
local excitation event and/or the specific global
excitation event, in response to determining that
the local excitation event corresponds to the
specific global excitation event

FIG. 8

## INTERNATIONAL SEARCH REPORT

| | International application No. |
|---|---|
| | **PCT/CN2023/124791** |

| A. | CLASSIFICATION OF SUBJECT MATTER |
|---|---|

A61N1/362(2006.01)i

According to International Patent Classification (IPC) or to both national classification and IPC

| B. | FIELDS SEARCHED |
|---|---|

Minimum documentation searched (classification system followed by classification symbols)

IPC: A61N

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 心, 除颤, 起搏, 电极, 脉冲, 发放, 时间, 检测, heart, defibrillation, pacing, electrode, pulse, delivery, time, detect

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 105102060 A (MEDTRONIC INC.) 25 November 2015 (2015-11-25) description, paragraphs 0001-0197, and figures 1-13 | 1-36 |
| Y | CN 217472580 U (HEYUAN MEDICAL DEVICES (SHANGHAI) CO., LTD.) 23 September 2022 (2022-09-23) description, paragraphs 0001-0138, and figures 1-3 | 1-36 |
| A | CN 112914717 A (SHAOXING MEIO MEDICAL TECHNOLOGY CO., LTD.) 08 June 2021 (2021-06-08) entire document | 1-36 |
| A | CN 111770724 A (MEDTRONIC INC.) 13 October 2020 (2020-10-13) entire document | 1-36 |
| A | CN 109247012 A (MEDTRONIC INC.) 18 January 2019 (2019-01-18) entire document | 1-36 |
| A | US 7751888 B1 (PACESETTER INC.) 06 July 2010 (2010-07-06) entire document | 1-36 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **15 December 2023** | **21 December 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2023/124791**

| Patent document cited in search report | | | Publication date (day/month/year) | Patent family member(s) | | | Publication date (day/month/year) |
|---|---|---|---|---|---|---|---|
| CN | 105102060 | A | 25 November 2015 | US | 2017043174 | A1 | 16 February 2017 |
| | | | | US | 10265534 | B2 | 23 April 2019 |
| | | | | WO | 2014120769 | A1 | 07 August 2014 |
| | | | | US | 2014214104 | A1 | 31 July 2014 |
| | | | | US | 9072914 | B2 | 07 July 2015 |
| | | | | US | 8744572 | B1 | 03 June 2014 |
| | | | | US | 2021299444 | A1 | 30 September 2021 |
| | | | | US | 2015297905 | A1 | 22 October 2015 |
| | | | | US | 9492677 | B2 | 15 November 2016 |
| | | | | US | 2019247673 | A1 | 15 August 2019 |
| | | | | US | 11033743 | B2 | 15 June 2021 |
| | | | | EP | 2950881 | A1 | 09 December 2015 |
| | | | | EP | 2950881 | B1 | 06 December 2017 |
| CN | 217472580 | U | 23 September 2022 | | None | | |
| CN | 112914717 | A | 08 June 2021 | | None | | |
| CN | 111770724 | A | 13 October 2020 | WO | 2019168772 | A1 | 06 September 2019 |
| | | | | EP | 3758599 | A1 | 06 January 2021 |
| | | | | US | 2019262619 | A1 | 29 August 2019 |
| | | | | US | 10561847 | B2 | 18 February 2020 |
| CN | 109247012 | A | 18 January 2019 | WO | 2018209078 | A1 | 15 November 2018 |
| | | | | US | 2018326215 | A1 | 15 November 2018 |
| | | | | US | 10881862 | B2 | 05 January 2021 |
| | | | | EP | 3621687 | A1 | 18 March 2020 |
| US | 7751888 | B1 | 06 July 2010 | | None | | |

Form PCT/ISA/210 (patent family annex) (July 2022)